# EUROPEAN PATENT APPLICATION

(11) **EP 1 908 846 A1**
(43) Date of publication of application: **09.04.2008**
(21) Application number: 06767415.0
(22) Date of filing: 27.06.2006
(51) Int. Cl.: C12P 13/04, C07C 229/36, C12N 1/00

(54) **METHOD OF PRODUCING COMPOUND HAVING ANTI-HCV ACTION**

(30) Priority: 28.06.2005 JP 2005188765
(71) Applicant: CHUGAI SEIYAKU KABUSHIKI KAISHA, Tokyo, 115-8543 (JP)
(72) Inventor: AOKI, Masahiro CHUGAI SEIYAKU KABUSHIKI KAISHA, Kanagawa 247-8530 (JP); NAGAHASHI, Yoshie CHUGAI SEIYAKU KABUSHIKI KAISHA, Kanagawa 247-8530 (JP); KATO, Hideyuki CHUGAI SEIYAKU KABUSHIKI KAISHA, Kanagawa 247-8530 (JP); ITO, Tatsuya CHUGAI SEIYAKU KABUSHIKI KAISHA, Kanagawa 247-8530 (JP); MASUBUCHI, Miyako CHUGAI SEIYAKU KABUSHIKI KAISHA, Kanagawa 247-8530 (JP); OKUDA, Toru, Koza-gun, Kanagawa 253-0111 (JP)
(74) Representative: HOFFMANN EITLE
(86) International application number: PCT/JP2006/312798
(87) International publication number: WO 2007/000994

(57) **Abstract**

There is provided a convenient and inexpensive method of producing a compound which has a high activity of inhibiting replication of hepatitis C virus (HCV) and is useful for preventing and treating a liver disease caused by HCV infection.

It is a method of biologically producing a compound represented by the formula (1): 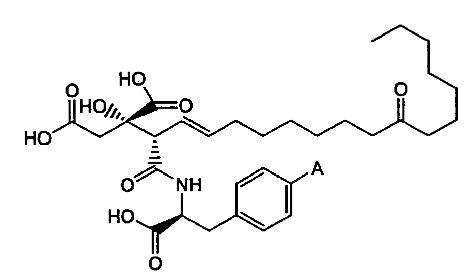 [wherein A represents a hydrogen atom, a straight or branched alkyl group having 1 to 8 carbon atoms, or the like], or a pharmaceutically acceptable salt thereof, a method comprising adding an amino acid derivative represented by the formula (3): 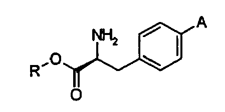 [wherein A has the same meaning as defined for the compound of the above formula (1); and R represents a hydrogen atom, a straight or branched alkyl group having 1 to 8 carbon atoms, or the like],
or a salt thereof into the fungal mycelium or the culture medium/culture broth of a filamentous fungal strain having an ability to produce the compound represented by the formula (2): 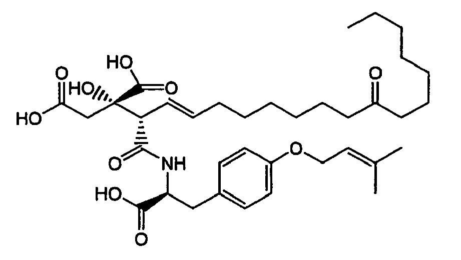 to thereby cause the fungal strain to directly produce the compound of the formula (1).

SEQUENCE LISTING

<110>  CHUGAI SEIYAKU KABUSHIKI KAISHA

<120>  Process for preparing a compound having an anti-HCV activity

<130>  FP2807PCT

<150>  JP2005-188765
<151>  2005-06-28

<160>  11

<170>  PatentIn version 3.1

<210>  1
<211>  20
<212>  DNA
<213>  Artificial sequence

<220>
<223>  primer

<400>  1
tcctccgctt attgatatgc                                                    20

<210>  2
<211>  22
<212>  DNA
<213>  Artificial sequence

<220>
<223>  primer

<400>  2
ggaagtaaaa gtcgtaacaa gg                                                 22

<210>  3
<211>  20
<212>  DNA
<213>  Artificial sequence

<220>
<223>  primer

<400>  3
atgggtaagg argacaagac                                                    20

<210>  4
<211>  21
<212>  DNA
<213>  Artificial sequence

<220>
<223>  primer

<400>  4
ggargtacca gtsatcatgt t                                                  21

<210>  5
<211>  19
<212>  DNA
<213>  Artificial sequence

<220>
<223>  primer

<400>  5

ctgaacgcct ctaagtcag 19

<210> 6
<211> 21
<212> DNA
<213> Artificial sequence

<220>
<223> primer

<400> 6
gagacaagca tatgactact g 21

<210> 7
<211> 26
<212> DNA
<213> Artificial sequence

<220>
<223> primer

<400> 7
tcaccaatca ctaacttcct cttccg 26

<210> 8
<211> 24
<212> DNA
<213> Artificial sequence

<220>
<223> primer

<400> 8
tgggatcctc agctttttct gcat 24

<210> 9
<211> 1904
<212> DNA
<213> Fusarium sp. F1476

<400> 9
ccccaacgac agactctcgc agtgcgaggg cgtggtaggg gttctcacct ttggaaagac 60

gggtcgagca ggcttcggcc tgccgatgcg tctaagtcga gggtgttcag ggtaggcagg 120

tcagacttgg tggaatcggg aatcggttcg agacgggttg cggtcgtggg atgaggttga 180

tttaaagttg cggaggtgcg ggtctgcagg gtaggcagga gtgccagagt cgagttgaga 240

gcccctctgg gacgaatagg tgatggtatg ggccggctgc agggtaggcg gggattgtct 300

tggtcgagct ggttgtcgtc cgggtggtga aaggcgatcg gggtcactgc agggtaggca 360

gaaatggctt ggtgggatgc ttggacggaa gaggcgccgg tctcctgccc aggtcgtgtc 420

gtgggaaacc ggtgcggaat ggtctggcgg tccggtggtc gtgagcacat ttttaaaatc 480

cgccatacaa atgaattttg cggaaaatca aagttggccc gcgagacggc tcgggcgagc 540

ggccggctgg gagagccccg gaggacctat gagagaaacg gcgccgggag gggcccaagg 600

agagctgcag ggtaggcagc cccaggttgg gcgtggaggc tcggtgctct tgtccttttg 660

ttgctagctt tcgatgtccg cattcactcg gtcgtctggt cggtcgtccg gctggtcagg 720

cagaaagtca ccctgacact cgtcagtcac gagctcatgc gttccgtggg aggtcacgtg 780

atcggtcacg tgcttttgta tatagctgat ttaactgatg gaagtgtctt agtcagcaaa 840

aaataactaa ttacaatatg attatcgagt tgttgacgga ttttgcctat gtgtaaatgc 900

ggagatgttg agaaaggtac gggaaattca aaaccaccaa ccttgtgacc ggaaacgcag 960

ctttgcgtgt gaccatggat gcagaaaaag ctgacgatcc caaaacacct ttccttttcc 1020

ccgtacgacg acggtcgagg cgtgaggttg ttttcgtgtc gatgtcggac tgatttaagg 1080

taggcaggat caacttcttg cctacccggt agctgagatt caaaacgacc tggcgcccgc 1140

cacagacctc gcccgaggtg gtgagatata tcattcggat acgccttggc ctgttctatg 1200

tgcctaggca ctcttgtgga aaccagtgcg aaatggtttt gcggtttggt ggtcgtgagc 1260

acatttttaa aatttcccat acaaatgaat tttgcggaaa ataaaaagtg gcccacgagc 1320

ccgatctggc gtgcggccga ctaaaacatt ctcggagggt atatgagagg ggagcaaagc 1380

agcccggccc gaaagggtgt ggacaaagct gcggcgcgaa cctccgtacc tgatcttgat 1440

agccgaccac tgtggcttcc ccggcgtgca ggcgtctttc ggacaaaccc ccggaagagg 1500

aagttagtga ttggtgaccc ccgtgaagcg tctggaataa cacgcgtagc ggggaccaag 1560

tccttcaacc agcctgaggc ataccaccga ggctgctggg ggcactgggc tctgtggatg 1620

gctggccact ggctagtact gaaacttagc acatcggggg aaaccacccg ctgctgaagc 1680

cctcgcggca gacgcagcgg gcgcggtcct ccaccatggg ctcctaagcc acagcaccct 1740

tcggggctcg ctgcggcgga cggtagccct ggaaaaccca gagtgggaaa gcggaacgcc 1800

ttgcgaggcg cgactggccc tgccggaagc gctgagcttg gttcagcccg gcgaaaggtg 1860

taaaaacctc tagctgctgt aactagtgag ctctccggcg cctt 1904

<210> 10
<211> 637
<212> DNA
<213> Fusarium sp. F1476

<400> 10
gtcgactctg gcaagtcgac cactgtgagt actaccctca atgaccttgc ttatcagcag 60

tcatcaaccc cccatacgtg gcggggtaat ttcaacttga atattggctg acaaaattgc 120

atagaccggt cacttgatct accagtgcgg tggtatcgac aagcgaacca tcgagaagtt 180

cgagaaggtt ggtttccatt ttcctcgatc gcacgccctc tgcccaccga tcaatcaccc 240

gaatccgtct tacgacgact gaatatgcgc ctgttacccc gctcgagtac aaaattttgc 300

ggttcaatcg taatttttgg tggggcttat accccgctac tcgagtgaca ggcgtttgcc 360

ctttcccaca aaatcatctc ttgcgcatca cgtgtcaaac agtcactaac cacccgacaa 420

taggaagccg ccgagctcgg taagggttcc ttcaagtacg cctgggttct tgacaagctc 480

ataggccgag cgtgagcgtg gtatcaccat cgatatcgcc ctctggaagt tcgagactcc 540

tcgctactat gtcaccgtca ttggtatgtt gtcaatcgct tacactcgtt accttcttat 600

gccaaacatg tgttccagac gctcccggtc caccgta 637

<210> 11
<211> 461
<212> DNA
<213> Fusarium sp. F1476

<400> 11
agtttacaac tcccaaaccc ctgtgaacat acctatacgt tgcctcggcg gatcagcccg 60

cgccccgtaa aacgggacgg cccgcccgag gacccctaaa ctctgttttt agtggaactt 120

ctgagtaaaa caaacaaata aatcaaaact ttcaacaacg gatctcttgg ttctggcatc 180

gatgaagaac gcagcaaaat gcgataagta atgtgaattg cagaattcag tgaatcatcg 240

aatctttgaa cgcacattgc gcccgccagt attctggcgg gcatgcctgt tcgagcgtca 300

tttcaaccct caagcccagc ttggtgttgg gactcgcggt aacccgcgtt ccccaaatcg 360

attggcggtc acgtcgagct tccatagcgt agtaatcata cacctcgtta ctggtaatcg 420

tcgcggccac gccgtaaaac cccaacttct gaatgttgac c 461

## Description

### Technical Field

The present invention relates to a method of producing a compound which has a high replication inhibitory activity against hepatitis C virus (HCV), and is useful for the prevention and treatment of liver diseases caused by viral infections, particularly by HCV infection.

### Background Art

It is estimated that there are 100 to 200 million patients infected with HCV around the world, and 2 million or more of such patients in Japan. About 50% of these patients progress to chronic hepatitis, and about 20% of them develop hepatic cirrhosis and hepatic cancer thirty years or more after the infection. Approximately 90% of the cases of hepatic cancer are said to be caused by hepatitis C. In Japan, more than 20,000 patients die each year from liver cancer concomitant to HCV infection.

HCV was discovered in 1989 as the primary causative virus of post-transfusion non-A, non-B hepatitis. HCV is an RNA virus having an envelope, and its genome is composed of a single-stranded (+)RNA. The virus is classified as belonging to the genus *Hepacivirus* of the Flaviviridae family.

Since HCV avoids the host's immune mechanism for unknown reasons, there are many cases in which sustained infection comes into effect even when adults having the immune mechanism fully developed are infected with the virus, and the infection then progresses to chronic hepatitis, hepatic cirrhosis and hepatic cancer. Thus, it is known that a large number of patients suffer from the recurrence of hepatic cancer because of the inflammation continually occurring at non-cancerous sites, even though the lesions are surgically extirpated.

Therefore, it is desirable to establish an effective therapeutic method for hepatitis C, and inter alia, besides the symptomatic therapy which suppresses inflammation through the use of anti-inflammatory drugs, there is a strong demand for the development of a drug alleviating or eradicating HCV in the liver which is the affected site.

Currently, the only therapeutic method known to be effective for eliminating HCV is interferon therapy. However, interferon is effective for only about one-third of all the patients. In particular, the efficacy of interferon against HCV genotype 1b is very low. Thus, there is a strong demand for the development of an anti-HCV drug that can be used in place of or in combination with interferon.

In recent years, ribavirin (1-β-D-ribofuranosyl-1H-1,2,4-triazole-3-carboxamide) has been commercially available as a therapeutic drug for hepatitis C to be used in combination with interferon; however, its efficacy is still low, and new hepatitis C therapeutic drugs are desired. Furthermore, although attempts have been made to eliminate the virus by enhancing the patient's immunity through the use of interferon agonists, interleukin-12 agonists and the like, no drug has been found to be effective.

Ever since the cloning of HCV gene, molecular biological analyses on the mechanisms and functions of viral genes, the functions of various viral proteins and the like have rapidly progressed, but the mechanisms for virus replication within host cells, sustained infection, pathogenicity and the like have not yet been fully elucidated, and at present, a reliable testing system for HCV infection using cultured cells has not been constructed. Thus, alternative virus assay methods which use other closely related viruses have to be used in evaluating anti-HCV drugs.

In recent years, however, it has become possible to observe HCV replication in vitro using a non-structural domain portion of HCV. Thus, anti-HCV drugs can now be evaluated easily by the replicon assay method (Non-Patent Document 1). The mechanism of HCV RNA replication in this system is considered to be the same as that of the replication of full length HCV RNA genome that has infected hepatocytes. Therefore, this system can be said to be an assay system based on cells that are useful for identifying compounds which inhibit HCV replication.

The inventors of the present invention isolated the following compound from *Fusarium* sp. strain F1476 (hereinafter referred to as "strain F1476"), which belongs to filamentous fungi, and found that the compound and derivatives thereof have high inhibitory activity for HCV replication (Patent Document 1). In addition, the compound is disclosed in WO 98/56755 (Patent Document 2), and is known to have an antifungal activity and an inhibitory effect against immune response.

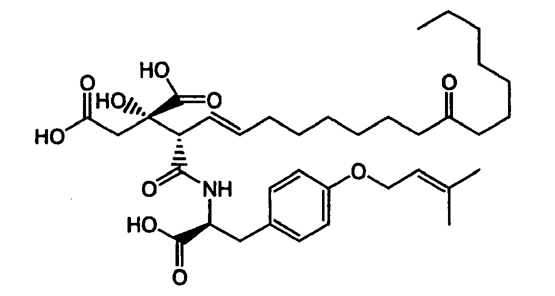

Furthermore, the inventors of the present invention developed a method for total synthesis of the compound and derivatives thereof, and found that various derivatives obtained by the method have likewise excellent HCV replication inhibitory activity (Patent Document 3). [Patent Document 1] International Publication No. WO 2004/071503 pamphlet
[Patent Document 2] International Publication No. WO 98/56755 pamphlet
[Patent Document 3] International Publication No. WO 2005/005372 pamphlet
[Non-Patent Document 1] V. Lohmann, et al., Science, 1999, Vol. 285, p. 110-113

### Disclosure of the Invention

### Problem to be solved the invention

As a result of intensive studies, the inventors of the present invention found a method of directly producing derivatives of the above-described compound using a fungal strain producing the compound ("strain F1476" and its mutants, etc.), by adding a salt of a specific amino acid derivative into the culture broth. Furthermore, the inventors of the present invention found the optimal culture conditions for directly producing the derivatives of the compound using said strain and the optimal salt of the amino acid derivative to be added, thus completing the present invention.

An object of the present invention is to provide a convenient and useful method of producing a compound which has high HCV replication inhibitory activity and thus is useful for the prevention and treatment of hepatic diseases caused by viral infection, particularly by HCV infection.

### Means for solving the problem

The present invention relates to a method of biologically producing a compound represented by the formula (1): 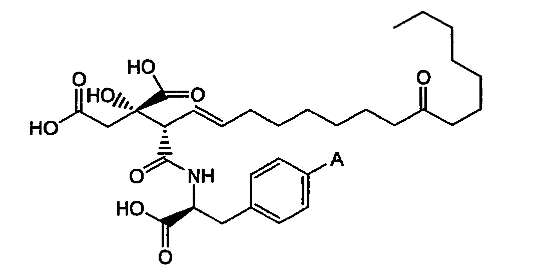 wherein A represents a hydrogen atom, a straight or branched alkyl group having 1 to 8 carbon atoms, a straight or branched alkenyl group having 2 to 8 carbon atoms, a straight or branched alkynyl group having 2 to 8 carbon atoms, -OR¹, an aryl group which may be substituted, or a heteroaryl group which may be substituted;

wherein R¹ represents a hydrogen atom, a straight or branched alkyl group having 1 to 8 carbon atoms, a straight or branched alkenyl group having 2 to 8 carbon atoms, a straight or branched alkynyl group having 2 to 8 carbon atoms, a cycloalkyl group having 3 to 8 carbon atoms, an aryl group which may be substituted, a heteroaryl group which may be substituted, an aralkyl group which may be substituted, or a heteroarylalkyl group which may be substituted,
or a pharmaceutically acceptable salt thereof, the method comprising adding an amino acid derivative represented by the formula (3):

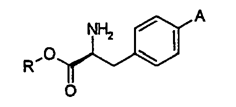

wherein A has the same meaning as defined for the compound of the above formula (1); and R represents a hydrogen atom, a straight or branched alkyl group having 1 to 8 carbon atoms, a straight or branched alkenyl group having 2 to 8 carbon atoms, or a straight or branched alkynyl group having 2 to 8 carbon atoms,
or a salt thereof into a fungal mycelium or a culture medium/culture broth of a filamentous fungal strain having an ability to produce a compound represented by the formula (2):

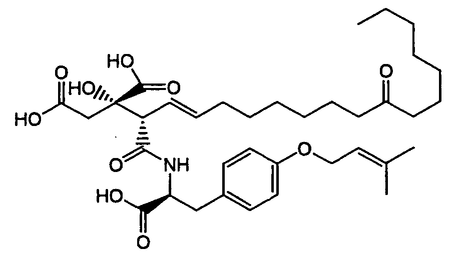

to thereby cause the fungal strain to directly produce the compound of the above formula (1).

Furthermore, the present invention relates to the production method described above, wherein R of the amino acid derivative of the formula (3) or a salt thereof is a straight or branched alkyl group having 1 to 4 carbon atoms, and particularly a methyl group.

The present invention also relates to the production method described above, wherein the amino acid derivative of the formula (3) or a salt thereof is a hydrochloride salt of the compound of the formula (3).

The present invention also relates to the production method described above, wherein A is a phenyl group which may be substituted, or a straight or branched alkynyloxy group having 2 to 8 carbon atoms.

The present invention also relates to the production method described above, wherein the fungal strain producing the compound of the formula (2) is strain F1476 or a strain taxonomically or genetically equivalent to said strain and particularly *Fusarium* sp. strain F1476 or a mutant strain thereof.
The present invention also relates to the production method described above, wherein the fungal strain producing the compound of the formula (2) is a strain having at least one feature selected from the following a) and b), or a mutant strain thereof:
a) the strain belongs to *Fusarium incarnatum,* or represents a form equivalent to that of *Fusarium incarnatum*;
b) the nucleotide sequence of ITS region of the strain is 99% or more homologous to that of *Fusarium* sp. strain F1476.

Furthermore, the present invention relates to the use of an amino acid derivative represented by the formula (3) : 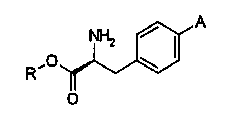 wherein A has the same meaning as defined for the compound of the above formula (1); and R represents a hydrogen atom, a straight or branched alkyl group having 1 to 8 carbon atoms, a straight or branched alkenyl group having 2 to 8 carbon atoms, or a straight or branched alkynyl group having 2 to 8 carbon atoms,
or a salt thereof as an additive for culture medium.

The present invention relates to an amino acid derivative represented by the formula (3'): 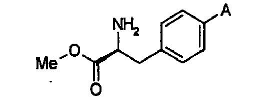 wherein A has the same meaning as defined for the compound of the above formula (1),
or a salt thereof.

Furthermore, the present invention relates to a medium additive containing an amino acid derivative represented by the formula (3): 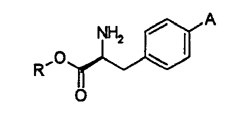 wherein A has the same meaning as defined for the compound of the above formula (1); and R represents a hydrogen atom, a straight or branched alkyl group having 1 to 8 carbon atoms, a straight or branched alkenyl group having 2 to 8 carbon atoms, or a straight or branched alkynyl group having 2 to 8 carbon atoms,
or a salt thereof.

### Effect of the invention

The present invention provides a method by which a compound of the formula (1) that is effective in the treatment and prevention of HCV infection such as hepatitis C, or a pharmaceutically acceptable salt thereof can be conveniently and inexpensively produced.

### Brief Description of the Drawings

Fig. 1 is a diagram showing the UV spectrum of compound 1;
Fig. 2 is a diagram showing the MS/MS spectrum of a standard product of the compound 1;
Fig. 3 is a diagram showing examples of the morphological properties of the wet part of strain F1476 on SNA;
Fig. 4 is a diagram showing examples of the morphological properties of the fast part of strain F1476 on SNA;
Fig. 5 is a diagram showing the phylogenetic tree by neighbor-joining method of Internal transcribed spacer (ITS region/466 bp) of strain F1476;
Fig. 6 is a diagram showing the phylogenetic tree by maximum parsimony method of Internal transcribed spacer (ITS region/466 bp) of strain F1476;
Fig. 7 is a diagram showing the phylogenetic tree by neighbor-joining method of Translation elongation factor 1-alpha (TEF 1-α/677 bp) region of strain F1476;
Fig. 8 is a diagram showing the phylogenetic tree by maximum parsimony methodof Translation elongation factor 1-alpha (TEF 1-α/677 bp) region of strain F1476;
Fig. 9 is a diagram showing the phylogenetic tree by neighbor-joining method of Intergenic spacer (IGS/2358 bp) of strain F1476;
Fig. 10 is a diagram showing the phylogenetic tree by maximum parsimony method of Intergenic spacer (IGS/2358 bp) of strain F1476; and
Fig. 11 is a diagram showing the MS/MS spectrum of a sample solution obtained by culturing *F. incarnatum* CBS 678.77.

### Best Mode for Carrying Out the Invention

In the present specification, a straight or branched alkyl group means a saturated hydrocarbon group having a predetermined number of carbon atoms. Examples of the straight or branched alkyl group having 1 to 8 carbon atoms include methyl, ethyl, n-propyl, i-propyl, n-butyl, sec-butyl, t-butyl, n-pentyl, t-pentyl, isopentyl, neopentyl, n-hexyl, isohexyl, neohexyl, 3-methylpentyl, n-heptyl, isoheptyl, 3-methylhexyl, 5-methylhexyl, 1,1-dimethylpentyl, 2,2-dimethylpentyl, 4,4-dimethylpentyl, 3-ethylpentyl, n-octyl, isooctyl, 3-methylheptyl, 2,2-dimethylhexyl, and the like.

A straight or branched alkenyl group means a hydrocarbon group which has a predetermined number of carbon atoms, and has at least one double bond. Examples of the straight or branched alkenyl group having 2 to 8 carbon atoms include vinyl, 1-propenyl, allyl, 1-butenyl, 2-butenyl, 2-methyl-1-propenyl, 1-methyl-1-propenyl, 1-pentenyl, 2-pentenyl, prenyl, 1-hexenyl, 2-hexenyl, 5-hexenyl, 4-methyl-3-pentenyl, 1-heptenyl, 6-heptenyl, 1-octenyl, 2-octenyl, 7-octenyl, and the like.

A straight or branched alkynyl group means a hydrocarbon group which has a predetermined number of carbon atoms, and has at least one triple bond. Examples of the alkynyl group having 2 to 6 carbon atoms include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 2-heptynyl, 3-heptynyl, 4-heptynyl, 5-heptynyl, 6-heptynyl, 1-octynyl, 2-octynyl, 4-octynyl, and the like.

Further, a cycloalkyl group means a cyclic saturated hydrocarbon group having a predetermined number of carbon atoms. Examples of the cycloalkyl group having 3 to 8 carbon atoms include a cyclopropyl group, a cyclobutyl group, a cyclopentyl group, a cyclohexyl group, a cycloheptyl group, and the like.
An aryl group described in the present specification means a monocyclic or polycyclic hydrocarbon group having aromaticity. Specifically, groups derived from benzene, naphthalene, anthracene, fluorene and the like may be mentioned.

A heteroaryl group described in the present specification means a 4- to 6-membered monocyclic or 7- to 10-membered bicyclic group (preferably, a monocyclic group) which has aromaticity, and contains 1 to 4 (preferably, 1 or 2) heteroatoms independently selected from a nitrogen atom, a sulfur atom and an oxygen atom as the ring member. Specifically, groups derived from furan, thiophene, pyrrole, pyrazole, pyridine, thiazole, imidazole, pyrimidine, indole, quinoline, oxazole, isoxazole, pyrazine, triazole, thiadiazole, tetrazole, pyrazole, and the like may be mentioned.

An aralkyl group described in the present specification means the aforementioned straight or branched alkyl group substituted with the aforementioned aryl group, and specifically, a benzyl group, a phenethyl group and the like may be mentioned.

A heteroarylalkyl group described in the present specification means the aforementioned straight or branched alkyl group substituted with the aforementioned heteroaryl group.

A halogen atom in the present specification means fluorine, chlorine, bromine or iodine.

The pharmaceutically acceptable salt thereof as described in the present specification is not particularly limited as long as the salt is pharmacologically acceptable. For example, salts with mineral acids such as hydrochloric acid, sulfuric acid, nitric acid, phosphoric acid, and hydrobromic acid; salts with organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid and camphorsulfonic acid; salts with alkali metals or alkaline earth metals such as sodium, potassium and calcium; and the like may be mentioned.

A salt of the amino acid derivative of the formula (3) as described in the present specification is not particularly limited, as long as the salt does not substantially give adverse effects on the growth of the fungal strain or the production of the compound of the formula (1) by the fungal strain in the medium. For example, salts with mineral acids such as hydrochloric acid, sulfuric acid, nitric acid, phosphoric acid, and hydrobromic acid; salts with organic acids such as acetic acid, tartaric acid, lactic acid, citric acid, fumaric acid, maleic acid, succinic acid, methanesulfonic acid, ethanesulfonic acid, benzenesulfonic acid, trifluoroacetic acid, toluenesulfonic acid, naphthalenesulfonic acid and camphorsulfonic acid; salts with alkali metals or alkaline earth metals such as sodium, potassium and calcium; ammonium salts and the like may be mentioned, and preferably salts with hydrochloric acid, trifluoroacetic acid and sodium may be mentioned.

A fungi having an ability to produce the compound represented by the formula (2) of the present invention may be any species, as long as the fungi can produce the compound represented by the formula (2) even in a small amount in the medium, and there may be mentioned, for example, *Fusarium* sp. strain F1476 (hereinafter, referred to as "strain F1476") (WO 04/071503) or a fungal strain which is taxonomically or genetically equivalent thereto, and mutant strains of these strains, *Aureobasidium sp.* strain TKR2449 (WO 98/056755) or a strain which is taxonomically or genetically equivalent thereto, and mutant strains of these strains. The fungi is preferably strain F1476 or a strain taxonomically or genetically equivalent thereto, and mutant strains of these strains.

A "taxonomically equivalent fungal strain" according to the present invention refers to a fungal strain having taxonomically identical features. Specifically, the term refers to a fungal strain having physiological properties as shown below, which are equivalent to those of strain F1476, and having an ability to produce the compound represented by the formula (2) of the present invention.

A "genetically equivalent fungal strain" according to the present invention refers to a fungal strain having genetically equivalent features. Specifically, the term refers to a fungal strain having identity, or 98% or greater, preferably 99% or greater homology in the sequence of ribosomal DNA such as 5S, 5.8S, 18S or 28S rDNA possessed by the strain, and having an ability to produce the compound represented by the formula (2) of the present invention.

Whether or not the fungal strain has an ability to produce the compound represented by the formula (2), can be recognized by culturing the strain, separating the compound from the culture, further purifying the separated compound according to necessity, and analyzing the compound or purified product.

As the culture method, separation method and purification method, various culture methods and culture conditions which use media containing various nutrition sources and acceptable additives; various separation methods; and various purification methods to be described below, which methods are described for the method of producing the compound of the formula (1) of the present invention, can be employed.

Furthermore, the amino acid derivative represented by the formula (3) that is added during the production of the compound of the formula (1) is in principle not added during the process of culture. However, for the purpose of determining whether or not the strain has an ability to produce the compound represented by the formula (2), a prenyltyrosine derivative represented by the formula (4): 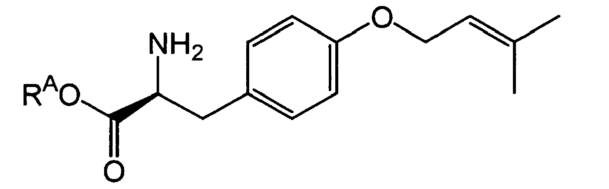 wherein R^{A} represents a hydrogen atom, or a straight or branched alkyl group having 1 to 4 carbon atoms, preferably a methyl group may be added, if necessary, as an amino acid derivative. The prenyltyrosine derivative represented by the formula (4) may be added as a free product, or as a hydrochloride, a trifluoroacetate salt or a sodium salt, but it is preferable that the derivative is added as a hydrochloride salt. The prenyltyrosine derivative represented by the formula (4) is preferably a hydrochloride salt of a compound having a methyl group for R^{A}. The form at the time of the addition (powder, dissolved in an organic solvent), the amount added and the culture conditions after the addition are the same as those for the amino acid derivative represented by the formula (3), which is to be added during the production of the compound of the formula (1).

As the analysis method to recognize the ability to produce the compound represented by the formula (2), there may be mentioned a method of comparing the data which is obtained from the compound obtained by culture of the fungal strain/separation/purification, according to well known measuring methods for, for example, the melting point, elemental analysis, NMR spectrum, IR spectrum, UV spectrum, MS spectrum, retention time in HPLC, optical rotation, X-ray structural analysis and the like, with the data of a compound of the formula (2) which is used as a standard product, and determining whether the data coincide or not.

In addition, these measuring methods may be appropriately combined and used for the determination, or measuring methods that are originally combined, for example, LC/MS, LC-MS/MS and high resolution MS analysis (hereinafter also referred to as TOF-MS) may also be employed.

Here, the compound used in the analysis may be a single product or a mixture of two or more compounds, and which is to be used can be appropriately selected in accordance with the analysis method.

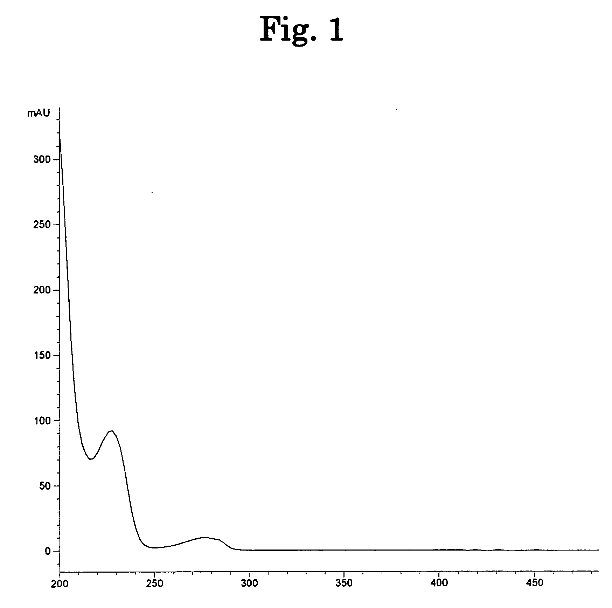

As the specific criteria for comparing the compound obtained by culture of the fungal strain /separation/purification, with the compound of the formula (2) which is used as a standard product, and determining whether or not the compounds coincide, for example, the following criteria may be mentioned:
1) In the MS spectrum, whether the molecular ion peak: 660 (M+H) and the fragment peak based on deprenylation: 592 are observed or not;
2) In the elemental analysis or TOF-MS, whether a molecular formula of C₃₆H₅₄NO₁₀ is derived or not;
3) Whether or not the UV spectrum shows a form as shown in Fig. 1 (compound 1: 100 mg/L; solvent: MeOH),
   that is, whether the compound shows a maximum absorption: λmax at 277 and 228 nm or not; and

4) In HPLC, whether peaks corresponding to any of the following i) to iii) are detected or not:
i) a peak having a retention time (RT) of 6.1 ± 0.1 minutes when the compound is analyzed under the analysis condition 1 of Example 2 of the present specification that will be described later;
ii) a peak having a retention time (RT) of 2.0 ± 0.1 minutes when the compound is analyzed under the analysis condition 3 of Example 4 of the present specification; and
iii) a peak having a retention time (RT) of 22.3 ± 0.1 minutes when the compound is analyzed under the analysis condition 5 of Test Example 1 of the present specification.

In addition, various data published in the literature may also be mentioned.

As described above, in the production method of the present invention, the above-described *Fusarium* sp. strain F1476 may be mentioned as the filamentous fungal strain having an ability to produce the compound represented by the formula (2), and the strain F1476 is a fungal strain belonging to *Fusarium incarnatum,* as will be described in Test Example 1 below.

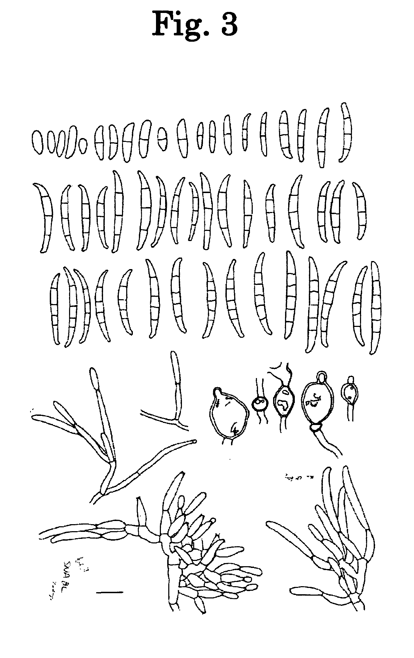

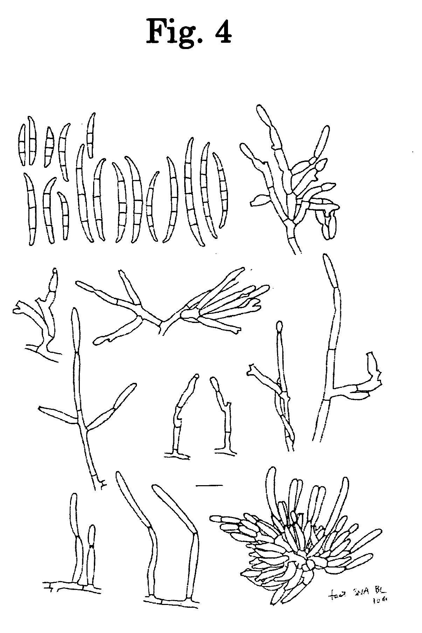

In the present invention, the phrase "having a morphology equivalent to that of *Fusarium incarnatum*" indicates that the fungal strain has all of the features of the following a) to c) :
a) The strain has polyphialide. Polyphialide refers to a morphology in which conidium is generated at the apex of a conidiogenous cell, subsequently a portion thereof is enlarged or grows, and the next generation conidium is generated at the tip of the portion.
b) The conidium is formed in clumps conglomerated with a phlegmatic temperament at the apex of a phialide, and typically becomes a lunate form, while occasionally taking an oblong shape or a cylindrical shape. The apex never spindles conspicuously. The conidium has well-defined podocytes at the base, or has a cut-off scar shape or an obtuse shape. The cut-off scar shape refers to the shape of the lower part left when the upper part of a conical body is cut along a cutting plane in the horizontal direction.
   In the determination of the morphology of such conidium, for example, reference may be made to the morphological properties and state of the conidium depicted, for example, in Ainsworth G.C., Kirk, P.M., Bisby, Guy Richard, Dictionary of the Fungi 9th Edition, CAB INTERNATIONAL, 2001, or in Test Example 1 of the present specification (Fig. 3 and Fig. 4).
c) The conidium has 0 to 5, preferably 2 to 4, septa. The conidium has a size corresponding to a length of 15.5 to 19.5 µm and a width of 2.5 to 3.5 µm for a biseptated conidium; a length of 15.5 to 37.5 µm and a width of 2.5 to 6.5 µm for a triseptated conidium; a length of 24.0 to 37.05 µm and a width of 3.0 to 6.0 µm for a quadriseptated conidium; and a length of 27.5 to 41.5 µm and a width of 3.0 to 6.0 µm for a pentaseptated conidium.

According to the present invention, the "ITS region" refers to a region combining the three parts of ITS1, 5.8S and ITS2 in the DNA sequence encoding the ribosomal RNA possessed by the fungal strain. If the sequences of the ITS region of two strains are 99% or more homologous, those strains can be determined to be allogeneic, and this is supported by, for example, the following Documents 1 to 3.
Document 1: Journal of Clinical Microbiology, vol. 37, 1985-1993 (1999)
Document 2: Mycoscience, vol. 40, 491-501 (1999)
Document 3: Journal of Clinical Microbiology, vol. 38, 1510-1515 (2000).

In particular, Document 3 has been established as a case example supportive of a filamentous fungi (the genus *Aspergillus*) such as strain F1476 (the genus *Fusarium*).

From the description in the above, as the strain that can be used as a filamentous fungal strain having an ability to produce the compound represented by the formula (2) for the production method of the present invention, there may be mentioned a fungal strain which either belongs to *Fusarium incarnatum,* or exhibits a morphology equivalent to that of *Fusarium incarnatum*; and/or has 99% or greater homology in the sequence of the ITS region in the strain's gene, with the sequence of strain F1476.

The strain F1476 that can be used in the present invention is a filamentous fungi separated from the fallen leaves collected at the southern slope of Kamakurayama in Kamakura city on January 24, 2000, by a washing and filtration method carried out on February 29, 2000.

The physiological properties of the strain F1476 are as follows:
The growth temperature range is from 10 to 30°C, and preferably from 20 to 30°C.
The pH range allowing growth is from 3 to 11, and preferably from 5 to 7.
The strain F1476 is indicated as *Fusarium* sp. F1476, and was deposited with the International Patent Organism Depositary of the National Institute of Advanced Industrial Science and Technology on February 4, 2003, under the accession number of FERM BP-8290.

The taxonomical features of the strain F1476 are as follows. A fungal strain which is taxonomically equivalent to the strain F1476 of the present invention refers to a fungal strain having the following taxonomical features, and having an ability to produce the compound represented by the formula (2) of the present invention.

### General properties for culture

Growth on potato dextrose agar medium (PDA) is slow, the colonyreaches a diameter of 12 mm after 10 days under irradiation with near ultraviolet light at 25°C, and the growth rate is 1.5 to 1.6 mm/day. Dense hyphae are formed, with the surfaces being undulated and raised, and wet sporodochia are concentrated in the central part, while prominently fluffy masses of hyphae are occasionally formed on the periphery. The color tone is light orange (Apricot color, Light orange, Light Apricot to Apricot, Munsell 5YR7/6 to 7/10, Methuen 6A6 to 6B8), while the reverse side shows a color ranging from light orange to orange (light orange, bright reddish orange, Tiger Lily, Munsell 5-10YR7/10, Methuen 6B8 to 8A6).

The growth rate is slightly poor in dark, being 0.9 to 1.1 mm/day, and the color tone is lighter and beige (pale beige, Ivory, Munsell 10YR9/2, Methuen 4A3), while the reverse side color is light reddish yellow (light reddish yellow, Naples Yellow). However, the color tone of the hypha may occasionally become darker, ranging from ocher to grayish brown (gold, Golden Ocher, light grayish brown, Blond, Munsell 10YR5/4-8, Methuen 5E5-8). In this case, the reverse side color is dark yellowish brown (chestnut, dark yellowish brown, Burnt Umber, Munsell 10YR3/6).

Growth on malt extract agar medium (MA) is slow, and the colony reaches a diameter of 11 mm after 11 days under irradiation with near ultraviolet light, with the growth rate being 1.0 mm/day. Dense hyphae are formed, and the surface is raised and exhibits a wooly or felt texture. The color tone is light orange (light orange, Light Apricot, Munsell 5YR8/6, Methuen 6A6), and the reverse side shows a bright orange color (bright orange, Nasturtium Orange, Munsell 5YR7/12, Methuen 6A7). The color tone is paler in dark.

Growth on oatmeal extract agar medium (OA) is fast, and the colonyreaches a diameter of 21 mm after 10 days under irradiation with near ultraviolet light, with the growth rate being 3.7 mm/day. The organism presents granular colonies which are flat, but have a large number of sporodochia densely concentrated in the central part. The color tone is light yellowish orange (light yellowish orange, Maize, Munsell 5YR7/8, Methuen 6B6), and the reverse side has the same color. The color tone is paler in dark.

Growth on synthetic nutrient agar medium (SNA) is slow, and the colony reaches a diameter of 13 mm after 7 days under irradiation with near ultraviolet light, with the growth rate being 1.6 mm/day. The strain forms plane and thin colony, and the colony have a felt or wooly texture, and have wet sporodochia sporadically present in the central part. The color tone is beige (pale beige, Ivory, Munsell 10YR9/2, Methuen 4A3), and the reverse side has the same color. Similar growth is observed in dark.

Growth on Miura's medium agar (LCA) is fast, and the colony reaches a diameter of 25 mm after 10 days under irradiation with near ultraviolet light, with the growth rate being 3.4 mm/day. The strain forms plane and thin colony, and the colony have a granular or fluffy texture, and have moist sporodochia sporadically present in the central part. The color tone is beige (pale beige, Ivory, Munsell 10YR9/2, Methuen 4A3), and the reverse side has the same color. Similar growth is observed in dark.

The growth temperature range is from 10 to 30°C, and the optimum growth temperature is from 20 to 30°C.
The growth pH range is pH 3 to 11, and the optimum pH is pH 5 to 7.

### Morphological properties

On SNA, conidiophores rise vertically primarily from aerial hyphae and branch off, and phialides are verticillate directly at the branches or the conidiophores, resulting in the formation of a complex conidiogerous structure. Phialides occasionally grow independently on aerial hyphae. Conidial structures may also be formed from hyphae groveling on the surface of agar. The conidiophores are 10 to 30 µm in length. The phialides are cylindrical and usually have a prominent cup-like structure on the apex, and the size is from 4.0 to 20.0 × 2.5 to 3.0 µm. The conidium is formed in clumps conglomerated with a phlegmatic temperament at the apex of a phialide, and typically becomes a lunate form, while occasionally taking an oblong shape or a cylindrical shape. The apex never spindles conspicuously. The conidium has well-defined podocytes at the base, or has a cut-off scar shape or an obtuse shape. Typically, the conidium has 2 to 4 septa, but may also have no septum, or have 1 or 5 septa. The size is 19.3 × 3.8 µm on average, with L/W being 5.2. Chlamydospores are not observed.

### Identification

A complex, tufted conidium structure formed from light-colored hyphae occasionally form conidiophores, and the conidium is of the phialo type which sporulates endogenously from the apex of a cylindrical phialide. The conidium is light-colored, and is of 2 to 5 cells having a characteristic boat-like or crescent shape. On the basis of the aforementioned morphological properties, this strain F1476 is determined to belong to the genus *Fusarium,* an imperfect fungi. Therefore, this fungal strain was identified as *Fusarium* sp. strain F1476.
References in which the methods of identification regarding the genus and species of the fungal strains belonging to the genus *Fusarium* are described are listed below.

### References

Gerlach, W. and Nirenberg, H. 1982. The genus Fusarium - a pictorial atlas. Mitt. Biol. Bundesanst. Land- u. Forstwirtsch. Berlin-Dahlem 209:1-406.
Booth, C. 1971. The genus Fusarium. CMI, Kew, Surrey, 237pp.
Carmichael, J.W., Kendrich, B., Conners, I.L. and Sigler, L. 1980. Genera of Hyphomycetes. University of Alberta Press, Edmonton.
Gams, W. 1971. Cephalosporium-artige Schimmelpilze (Hyphomycetes) Gustav Fischer Verlag, Stuttgart. 262pp.

Furthermore, in the present invention, it is allowed to exclude *Fusarium incarnatum* strain CBS 678.77, if necessary, from the fungal strains having an ability to produce the compound represented by the formula (2).

In the present invention, the "mutant strain" refers to a naturally-occurring or artificial mutant strain having an ability to produce the compound represented by the formula (2), such as the above-described strain F1476 or strain TKR2449, and a fungal strain having an ability to produce the compound represented by the formula (2).

In general, it will be easy for those skilled in the art to produce, using known methods, a variety of mutant strains from a fungi having an ability to produce the compound represented by the formula (2), such as the above-described strain F1476 or strain TKR2449, the mutant strains including, for example, a mutant strain having an ability that is superior to that of the aforementioned fungi (for example, an ability to produce the compound represented by the formula (1) or the formula (2) of the present invention, etc.).

For instance, a mutant strain can be obtained by treating the spores or hyphae of the aforementioned fungi with a physical means such as the irradiation with γ-ray, X-ray or ultraviolet ray, or with a chemical substance such as nitrous acid, ethyl methanesulfonate, methyl methanesulfonate or 1-methyl-3-nitro-1-nitrosoguanidine, subsequently washing and diluting the treated spores or hyphae, cultivating them by applying on an agar medium such as a potato dextrose agar plate, and isolating the resulting mutant strain. Thereafter, each of the obtained strains is subjected to solid culture or liquid culture, and the culture broth is extracted with an appropriate solvent or the like, and then analyzed using analytical instruments such as HPLC and LC/MS, to evaluate the productivity of each strain for the compound. Thus, a variety of mutant strains, such as highly productive mutant strains, can be obtained.

The method of producing the compound of the present invention will be described in detail in the following.
The compound of the formula (1) of the present invention is represented by the formula shown in the above, and in the formula (1), A represents a hydrogen atom, a straight or branched alkyl group having 1 to 8 carbon atoms, a straight or branched alkenyl group having 2 to 8 carbon atoms, a straight or branched alkynyl group having 2 to 8 carbon atoms, -OR¹, an aryl group which may be substituted, or a heteroaryl group which may be substituted;

wherein R¹ represents a hydrogen atom, a straight or branched alkyl group having 1 to 8 carbon atoms, a straight or branched alkenyl group having 2 to 8 carbon atoms, a straight or branched alkynyl group having 2 to 8 carbon atoms, a cycloalkyl group having 3 to 8 carbon atoms, an aryl group which may be substituted, a heteroaryl group which may be substituted, an aralkyl group which may be substituted, or a heteroarylalkyl group which may be substituted.

R¹ is preferably a straight or branched alkynyl group having 2 to 8 carbon atoms, and particularly preferably a butynyl group such as 2-butyn-1-yl.

For the substituent of "the aryl group which may be substituted or the heteroaryl group which may be substituted" described above, a halogen atom, -OR², a straight or branched alkyl group having 1 to 8 carbon atoms, a straight or branched alkenyl group having 2 to 8 carbon atoms, a straight or branched alkynyl group having 2 to 8 carbon atoms, a cyano group, a nitro group, a trifluoromethyl group, an amino group which may be mono- or disubstituted with a straight or branched alkyl group, an acyl group, a straight or branched alkylsulfonyl group, a carbamoyl group, a straight or branched alkylthio group, a carboxyl group, a straight or branched alkylcarbonyl group, a formyl group, an aminosulfonyl group, and the like may be mentioned, and the aryl group or heteroaryl group may be mono-, di- or trisubstituted with these. Here, R² represents a hydrogen atom, a straight or branched alkyl group having 1 to 4 carbon atoms, or a trifluoromethyl group. The aforementioned substituent is preferably fluorine, a methoxy group, a methyl group or a dimethylamino group.
Preferred examples of the aryl group which may be substituted include a phenyl group which may be substituted with a straight or branched alkyloxy group having 1 to 8 carbon atoms, and a phenyl group which may be substituted with a halogen atom, and more preferably, a methoxyphenyl group and a fluorophenyl group may be mentioned.
Preferred examples of the heteroaryl group which may be substituted include a pyridyl group.

As A in the compound of the formula (1) of the present invention, there may be preferably mentioned
- OR¹; a phenyl group substituted with a halogen atom or a methoxy group; and a pyridyl group, and a butynyloxy group such as 2-butyn-1-yloxy is particularly preferable.

The amino acid derivative of the formula (3) of the present invention is a compound represented by the formula shown above, and in the formula (3), A has the same definition for A of the compound of the formula (1) described above.
Furthermore, as such amino acid derivative of the formula (3), there may be mentioned a free carboxylic acid wherein R is a hydrogen atom, and methyl or t-butyl ester of carboxylic acid wherein R is methyl or t-butyl. Among them, the amino acid derivative represented by the aforementioned formula (3'), wherein R is methyl, may be mentioned.

Furthermore, the amino acid derivative of the formula (3) of the present invention can be prepared, for example, as follows.

### Production Method-1

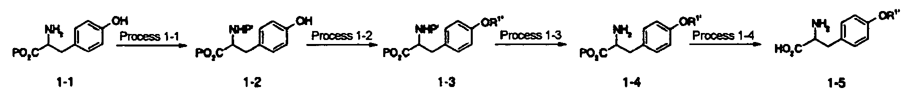

In the above formulae, P represents a straight or branched alkyl group having 1 to 8 carbon atoms, a straight or branched alkenyl group having 2 to 8 carbon atoms, a straight or branched alkynyl group having 2 to 8 carbon atoms, or a protective group for carboxyl group; P' represents a protective group for amino group; R¹' represents, as desired, a straight or branched alkyl group having 1 to 8 carbon atoms, a straight or branched alkenyl group having 2 to 8 carbon atoms, a straight or branched alkynyl group having 2 to 8 carbon atoms, a cycloalkyl group having 3 to 8 carbon atoms, an aryl group which may be substituted, a heteroaryl group which may be substituted, an aralkyl group which may be substituted, or a heteroarylalkyl group which may be substituted.

### Process 1-1

Compound 1-1 is a commercially available compound (L-tyrosine-t-butyl ester, L-tyrosine methyl ester, etc.), or a compound which can be easily synthesized from a commercially available compound (L-tyrosine, etc.), and when this is protected with a protective group for amino group, such as acetyl, trifluoroacetyl, t-butoxycarbonyl, benzyloxycarbonyl or 9-fluorenylmethylcarbonyl, the compound 1-2 can be obtained. As for the reaction conditions for this process, the conditions for the protective group for amino group as described in "Protective Groups in Organic Synthesis" by Theodora Greene, 1999, Wiley-Interscience, are used.

### Process 1-2

When the compound 1-2 is reacted with the above-defined R^{1,} which has been conjugated with a halogen atom or a leaving group such as mesylate or tosylate, at room temperature or under heating, preferably at room temperature, in a solvent such as diethyl ether, toluene, cyclohexane, acetone, dimethylformamide, dioxane, ethyl acetate or dimethyl sulfoxide, or a mixture thereof, in the presence of a base such as potassium carbonate, sodium hydroxide or sodium hydride, the compound 1-3 to which a desired group R^{1,} has been introduced can be obtained. Alternatively, the compound 1-3 can also be obtained by reacting the compound 1-2 with the above-defined R¹' which has been substituted with a hydroxyl group, under the conditions of Mitsunobu reaction.

### Process 1-3

The protective group P' for amino group of the compound 1-3 is deprotected to obtain the compound 1-4. For the reaction conditions for this process, the deprotection conditions for a protective group for amino group as described in "Protective Groups in Organic Synthesis" by Theodora Greene, 1999, Wiley-Interscience, are used.

### Process 1-4

The protective group P for carboxyl group of the compound 1-4 is deprotected to obtain the compound 1-5. For the reaction conditions for this process, the deprotection conditions for a protective group for carboxyl group as described in "Protective Groups in Organic Synthesis" by Theodora Greene, 1999, Wiley-Interscience, are used.

### Production Method-2

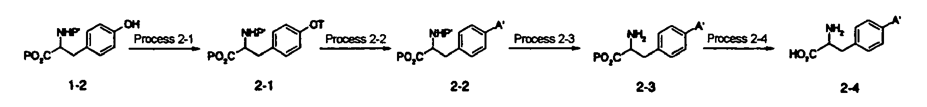

In the above formulae, P represents a straight or branched alkyl group having 1 to 8 carbon atoms, a straight or branched alkenyl group having 2 to 8 carbon atoms, a straight or branched alkynyl group having 2 to 8 carbon atoms, or a protective group for carboxyl group; P' represents a protective group for amino group; T represents a leaving group such as mesylate, toluenesulfonate or trifluoromethanesulfonate; A' represents an aryl group which may be substituted, a heteroaryl group which may be substituted, a straight or branched alkyl group having 1 to 8 carbon atoms, a straight or branched alkenyl group having 2 to 8 carbon atoms, or a straight or branched alkynyl group having 2 to 8 carbon atoms.

### Process 2-1

The compound 1-2 obtained in Process 1-1 is reacted with a compound which can be a leaving group such as methanesulfonyl chloride, toluenesulfonyl chloride or trifluoromethanesulfonic anhydride, at room temperature or under cooling, preferably under cooling, in a solvent such as diethyl ether, toluene, cyclohexane, acetone, dimethylformamide, dioxane, ethyl acetate or dimethyl sulfoxide, or a mixture thereof, in the presence of a base such as N,N-diisopropylethylamine, triethylamine, pyridine or 4-N,N-dimethylaminopyridine to obtain the compound 2-1 to which a leaving group T has been introduced.

### Process 2-2

The compound 2-1 is reacted with an aryl or heteroaryl boric acid derivative or an aryl or heteroaryl boric ester derivative, which has a desired aryl group or heteroaryl group, A', or a tetraalkyltin derivative or an alkenyl or alkynyltri-n-butyltin derivative having a desired alkyl group, alkenyl group or alkynyl group, A', at room temperature or under heating, preferably under heating, in a solvent such as diethyl ether, toluene, benzene, dimethylformamide, dioxane, ethyl acetate, acetonitrile or water, or in a mixture thereof, in the presence of a palladium catalyst such as palladium diacetate or tetrakis(triphenylphosphine) palladium, to obtain the compound 2-2.

### Process 2-3

The protective group P' for amino group of the compound 2-2 is deprotected to obtain the compound 2-3. For the reaction conditions for this process, the deprotection conditions for a protective group for amino group as described in "Protective Groups in Organic Synthesis" by Theodora Greene, 1999, Wiley-Interscience, are used.

### Process 2-4

The protective group P for carboxyl group of the compound 2-3 is deprotected to obtain the compound 2-4. For the reaction conditions for this process, the deprotection conditions for a protective group for carboxyl group as described in "Protective Groups in Organic Synthesis" by Theodora Greene, 1999, Wiley-Interscience, are used.

An amino acid derivative of the formula (3) wherein A is a group other than the groups described above, or a salt thereof can also be synthesized according to the above-described methods. Furthermore, an amino acid derivative of the formula (3) wherein R is a hydrogen atom, a straight or branched alkyl group having 1 to 8 carbon atoms, a straight or branched alkenyl group having 2 to 8 carbon atoms, or a straight or branched alkynyl group having 2 to 8 carbon atoms, or a salt thereof can be synthesized by subjecting the compound obtained by the above-described Production Methods 1 and 2 to a deprotection reaction of a carboxyl group and a reaction such as esterification, as necessary.

As the preferred compound of the formula (I) of th present invention, the following compounds may be mentioned.

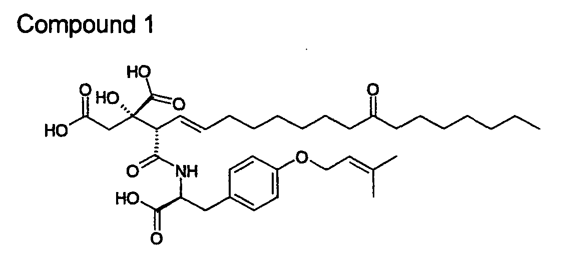

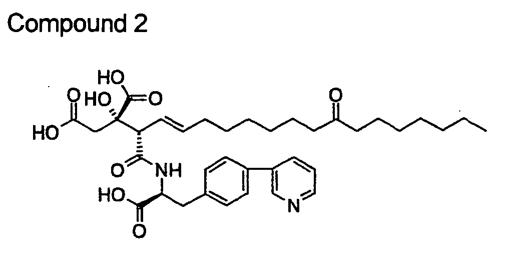

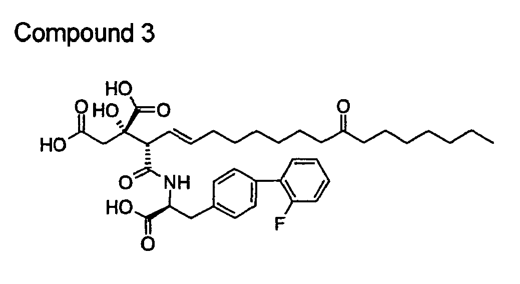

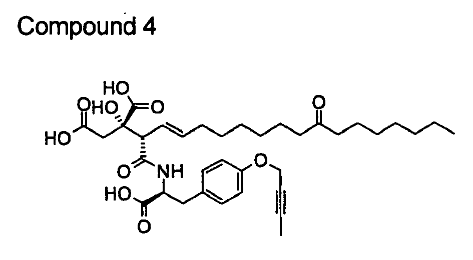

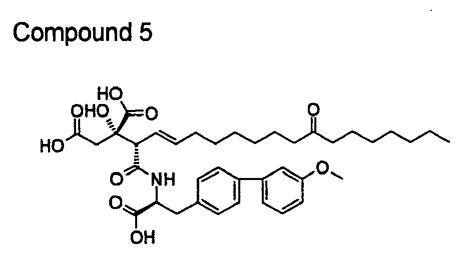

The method of producing the compound of the formula (1) of the present invention comprises culturing a fungal strain having an ability to produce the compound of the formula (2) discussed above in a culture medium which is prepared by adding an amino acid derivative of the formula (3) or a salt thereof to a medium (preferably, a culture broth) suitable for the growth of the fungal strain, and allowing the aforementioned strain to produce the desired compound of the formula (1).

The medium used for the culturing to produce the compound of the formula (1) of the present invention may be any medium containing sources of nutrients that the fungal strain having an ability to produce the compound of the formula (2) (for example, strain F1476 or a mutant strain thereof, etc.), and various synthetic or semisynthetic media, natural media and the like can all be used.

Among the sources of nutrients, examples of carbon sources include glucose, fructose, lactose, saccharose, maltose, xylose, sucrose, starch, dextrin, glycerin, molasses, starch syrup, oils and fats, organic acids, and the like. From the viewpoint of the efficiency in producing the compound of the formula (1), glucose, fructose, lactose and dextrin are preferred, and among them, lactose is more preferred.

Among the above sources of nutrients, examples of nitrogen sources include organic nitrogen compounds such as soybean flour, cottonseed flour, corn steep liquor, casein, peptone, yeast extract, meat extract, germs, Pharmamedia, urea and amino acids, inorganic nitrogen compounds such as ammonium salts, for example, ammonium nitrate and ammonium sulfate, and the like.

Among the sources of nutrients, examples of salts include inorganic salts such as sodium salts, potassium salts, calcium salts, magnesium salts and phosphate salts. These may be used individually, or may be used in appropriate combinations.

The above-described sources of nutrients can be used individually or in appropriate combinations.

The medium containing the above-mentioned sources of nutrients may be appropriately incorporated with heavy metal salts such as iron salts, copper salts, zinc salts and cobalt salts; vitamins such as biotin and vitamin B₁; and in addition to these, organic substances and inorganic substances that promote the production of the compound of the present invention by helping the growth of the fungi, as necessary.

The medium containing the above-mentioned sources of nutrients may also be further incorporated with defoaming agents or surfactants such as silicone oils and polyalkylene glycol ether, and the like, in addition to the aforementioned sources of nutrients, as necessary.

The culturing conditions can be appropriately selected within the scope where the fungal strain can favorably grow. Typically, culturing is conducted at pH 3 to 11 and at a temperature of 10 to 30°C for about 5 to 10 days. The above-described various culturing conditions can be appropriately selected in accordance with the type or properties of the fungi used, external conditions and the like, and optimal conditions can be selected.

To the thus-conditioned culture broth containing the fungal strain, the amino acid derivative of the formula (3) is added to produce the desired compound of the formula (1). That is, the amino acid derivative of the formula (3) or a salt thereof is added directly to the culture broth containing fungal mycelia during culture, or added, after the completion of culturing, to a suspension prepared by suspending the fungal mycelia separated from the culture broth by centrifugation or filtration in another liquid for culture, and the fungal mycelia are further cultured. The liquid for culture that can be used for the suspension of fungal mycelia is preferably the above-mentioned culture media or combinations thereof.

The amino acid derivative of the formula (3) or a salt thereof to be added to the culture medium is preferably a salt such as hydrochloride, trifluoroacetate or sodium salt. The aforementioned amino acid derivative may be added to the medium directly in a powder form, or after being dissolved in water or a water-soluble organic solvent such as ethanol. The amount to be added is preferably 50 to 2000 mg per 1 ml of the medium.

After the addition of the aforementioned amino acid derivative, the desired compound of the formula (1) can be produced by culturing at 25 to 30°C for 3 to 14 days, preferably for about 5 to 10 days, under shaking conditions or under aeration and stirring conditions.

When culturing the fungal strain which produces the compound of the present invention in the above-described medium containing the sources of nutrients, culturing methods that are generally used when performing the production of physiologically active substances by culturing a microorganism, such as solid culture methods and liquid culture methods, can be employed, and preferably, a liquid culture method can be used. When employing a liquid culture method, it is preferable to use glucose, fructose, lactose and dextrin as the source of carbon, which is one of the sources of nutrients, and among them, it is more preferable to use lactose.
Furthermore, as preferred liquid media for the liquid culture method, for example, a liquid medium 2 described in Example 5, a liquid medium 3 described in Example 7, and a liquid medium 5 described in Example 8 of the present specification may be mentioned.

According to the culturing methods described above, the compound of the present invention is accumulated in the culture broth. In the present invention, the compound accumulated in the culture broth can be separated therefrom by a known method and then further purified as necessary.

The above separation can be performed by extracting the whole culture broth with a non-hydrophilic organic solvent such as ethyl acetate, butyl acetate, chloroform, butanol or methyl isobutyl ketone, or a hydrophilic organic solvent such as methanol or acetone. Also, the culture broth may be separated into the solution portion of the culture broth and the fungal mycelium by filtration or centrifugation, and then the compound may be separated respectively from the solution portion of the culture broth and the fungal mycelium.

In order to separate the compound of the present invention from the culture broth separated as described above, a method of extracting the culture broth with the above-mentioned non-hydrophilic organic solvents may be employed, or a method of contacting the culture broth with an adsorptive carrier to adsorb the compound in the culture broth to the carrier, and then eluting the compound with a solvent, may also be employed.

As the carrier, for example, activated carbon, powdered cellulose, adsorptive resins and the like may be mentioned. The solvent can be appropriately used individually or in combination of two or more kinds, in accordance with the type, properties and the like of the carrier, and for example, there may be mentioned appropriate combinations of hydrous solutions of water-soluble organic solvents, such as hydrous acetone and hydrous alcohols. The water content in the hydrous solution can be appropriately selected in accordance with the type of the carrier or water-soluble organic solvent, or the like, and for example, the water content is 10 to 90% (V/V), and preferably 40 to 60% (V/V).

To separate the starting compound of the present invention from the fungal mycelia separated as described above, a method of extracting the compound with a hydrophilic organic solvent such as acetone or methanol can be employed.

According to the present invention, a crude extract of the compound of the present invention which has been thus separated from the culture broth, may be further subjected to a purification process, if necessary.

The purification can be performed according to the methods conventionally used in the separation and purification of oil-soluble, physiologically active substances, and as such methods, for example, column chromatography methods or high performance liquid chromatography methods, using a carrier such as silica gel, active alumina, activated carbon or adsorptive resins, and the like may be mentioned. In the case of employing a column chromatography method using silica gel as the carrier, for example, chloroform, ethyl acetate, methanol, acetone, water and the like may be mentioned as the eluent, and these may be used in combination of two or more kinds. Further, trifluoroacetic acid and acetic acid can be added to the eluent.

In the case of employing a high performance liquid chromatography method, for example, chemically bonded silica gel to which an octadecyl group, an octyl group, a phenyl group or the like has been bonded; polystyrene-based porous polymer gels; and the like may be mentioned as the carrier, while hydrous solutions of water-soluble organic solvents such as, for example, hydrous methanol and hydrous acetonitrile, may be used as the mobile phase. Furthermore, trifluoroacetic acid and acetic acid can be added to the mobile phase.

As described above, the method of producing the compound of the formula (1) of the present invention does not use complicated processes of chemical synthesis, but produces the desired compound of the formula (1) directly from the fungal mycelia, as compared with conventionally known production methods, and thus can produce the desired compound in a large amount, more conveniently than before.

In addition, when producing a compound of the formula (1) of the present invention, wherein A is a group other than a prenyloxy group, there are cases where the compound represented by the formula (2), for which the fungal strain originally has a capability for production, is obtained as a compound in addition to the desired compound. In those cases, purification is performed by the above-described methods conventionally used in the separation and purification of oil-soluble physiologically active substances, and the compound of the formula (1) can be obtained by separation from the compound represented by the formula (2). Furthermore, the compound of the formula (1) may also be more efficiently obtained by preparing a mutant strain of the fungal strain which can produce the compound represented by the formula (2), and selecting and using a fungal strain which does not produce the compound of the formula (2) unless prenyltyrosine represented by the formula (4) is added, or which has a lower ratio for the production of the compound of the formula (2) to the production of the compound of the formula (1), as compared to the ratio prior to the mutation.

Furthermore, in the method of producing the compound of the formula (1) of the present invention, it is permitted to exclude, if necessary, the compound of the formula (2) from the compound of the formula (1).

The compound of the formula (1) of the present invention thus obtained can be used directly, or as a prodrug or pharmaceutically acceptable salt thereof, as an active ingredient for a pharmaceutical composition, particularly a therapeutic agent for hepatic diseases caused by HCV.

### EXAMPLES

Hereinafter, the present invention will be specifically described with reference to Examples, but the present invention is not limited to them. In addition, amino acids 1 to 6 used and compounds 1 to 5 produced in the following respective Examples will be described later.

### Example 1

### Production of compound 2 by solid culture method

One loopful of microorganisms obtained from a slant culture of *Fusarium* sp. strain F1476 was inoculated into a 500 ml Erlenmeyer flask with baffles containing 100 ml of liquid medium 1 (2.0% glucose, 1.5% glycerol, 1.0% potato starch, 0.25% polypeptone, 0.35% yeast extract, 0.5% calcium carbonate, 0.3% sodium chloride, 0.005% zinc sulfate heptahydrate, 0.0005% copper sulfate pentahydrate, 0.0005% manganese chloride tetrahydrate, and 1.0% toasted soya bean), and cultured with shaking at 27°C for 3 days at 220 rpm, to obtain a seed culture broth. 2 ml of the seed culture broth was inoculated to a brown rice solid medium (5 g of germinated brown rice, 2 ml of desalted water) in 50 ml polypropylene tube. An aqueous solution of amino acid 1 was prepared to a concentration of 10 mg/ml and then sterilized by filtration. The solution was added to the polypropylene tube in an amount of 1 ml at the initiation of culture, and in an amount of 0.5 ml each at 48 hours and 96 hours after the initiation of culture, and stationary culture was performed at 27°C for 168 hours. To the obtained culture broth, 10 ml of methanol was added, and the mixture was stirred by shaking at 180 rpm for 20 minutes, and then centrifuged at 3000 rpm for 10 minutes. 500 µl of the obtained methanol extract was concentrated and dried, and then dissolved in 10 µl of dimethyl sulfoxide and 90 µl of methanol. The solution was analyzed under analysis condition A indicated in the following analysis conditions, and as a result, production of compound 2 was observed. The type of the amino acid added and the physicochemical properties of the produced compound are shown in Table 1. The produced compound 2 was analyzed under the conditions of LC/MS shown as the analysis condition 2, and identified on the basis that the M+H value and RT of mass spectrum coincided with the corresponding values of the standard compound.

Analysis condition A
Apparatus: LC-MS Agilent 1100 series ChemStations system, LCQ Navigator system
Column: Develosil Combi RP-5 (5 µm, 4.6 mm × 50 mm) (manufactured by Nomura Chemical Co., Ltd.)
Mobile phase: Solvent gradient elution shown below
Liquid A: water (0.01% trifluoroacetic acid)
Liquid B: acetonitrile (0.01% trifluoroacetic acid)

| Minute | % Liquid B |
|---|---|
| 0 | 15 |
| 0.7 | 15 |
| 1 | 35 |
| 5 | 55 |
| 6 | 82 |
| 7.2 | 98 |
| 7.6 | 98 |
| 8.2 | 15 |
| 8.8 | 15 |

Flow rate: 3 ml/min, Temperature: room temperature

**Table 1: Physicochemical properties of the compound produced from amino acid-added culture**

| Amino acid added | Compound produced | RT (min) | ESI positive mode (M+H) | Molecular weight |
|---|---|---|---|---|
| Amino acid 1 | Compound 2 | 4.3 ± 0.1 | 653 | 652 |

### Example 2

### Acquisition of strain F1476-C193

A spore suspension (number of spores: about 5x10⁶/ml) was prepared from *Fusarium* sp. strain F1476 (FERM BP-8290) grown on an MA slant medium (1.0% malt extract, 0.1% yeast extract, 0.1% bactosoytone, 1.0% glucose, 2.0% agar), using 4 ml of sterilized saline. 0.3 ml of the obtained spore suspension, 0.3 ml of 1-methyl-3-nitro-1-nitrosoguanidine prepared to a concentration of 3 mg/ml, and 2.4 ml of saline were mixed and shaken at 27°C at 127 rpm for 1 hour, to carry out a mutation treatment. 2 ml of the treated suspension was centrifuged at 14,000 rpm for 10 minutes, the supernatant was discarded, and the spores were washed with 2 ml of sterilized saline. The spores were then diluted and applied to potato dextrose agar plates (manufactured by Nihon Pharmaceutical Co., Ltd.) in an amount of 0.1 ml each. The spores were cultured at room temperature for 5 to 6 days, and the resulting colonies were spread over a potato dextrose agar slant medium, to obtain 140 candidate mutant strains. The obtained mutant strains were each suspended in sterilized water, and the suspension was inoculated to a brown rice solid medium (5 g of germinated brown rice, 2 ml of desalted water) in 50 ml of polypropylene tube, and subjected to stationary culture at 27°C for 192 hours. To the obtaind culture broth, 10 ml of methanol was added, and the mixture was stirred by shaking at 180 rpm for 20 minutes, and then centrifuged at 3000 rpm for 10 minutes. 500 µl of the obtained methanol extract was concentrated and dried, and then dissolved in 10 µl of dimethyl sulfoxide and 90 µl of methanol. The solution was analyzed under the analysis condition 1 described below, and high-producing strains for compound 1 were screened. As a result, a high-producing strain for compound 1, *Fusarium* sp. strain F1476-C193, could be obtained (Table 2) .

**Table 2: Productivity for compound 1 of strain F1476 and strain F1476-C193**

| Strain | Compound produced | Concentration (mg/L) |
|---|---|---|
| F1476 | Compound 1 | 12-160 |
| F1476-C193 | Compound 1 | 230-280 |

Physicochemical properties of compound 1
Molecular weight: 659
ESI (LC/MS positive mode) m/z = 660 (M+H)
Retention time (RT) under analysis condition 1: 6.1 ± 0.1 minutes

Analysis condition 1
Apparatus: HPLC Agilent 1100 series (ChemStations system) Column: Develosil Combi RP-5 (5 µm, 4.6 mm ×50 mm) (manufactured by Nomura Chemical Co., Ltd.)
Mobile phase: Solvent gradient elution shown below
Liquid A: water (0.01% trifluoroacetic acid)
Liquid B: acetonitrile (0.01% trifluoroacetic acid)

| Minute | % Liquid B |
|---|---|
| 0 | 15 |
| 0.7 | 15 |
| 1 | 35 |
| 5 | 55 |
| 6 | 82 |
| 7.2 | 98 |
| 7.6 | 98 |
| 8.2 | 15 |
| 8.8 | 15 |

Flow rate: 3 ml/min, Temperature: room temperature

### Example 3

### Production of compound 2, compound 3 and compound 4 by solid culture method

One loopful of microorganisms obtained from a slant culture of strain F1476-C193 was inoculated into a 500 ml Erlenmeyer flask with baffles containing 100 ml of liquid medium 1 (2.0% glucose, 1.5% glycerol, 1.0% potato starch, 0.25% polypeptone, 0.35% yeast extract, 0.5% calcium carbonate, 0.3% sodium chloride, 0.005% zinc sulfate heptahydrate, 0.0005% copper sulfate pentahydrate, 0.0005% manganese chloride tetrahydrate, and 1.0% toasted soya bean), and cultured with shaking at 27°C for 3 days at a shaking rate of 220 rpm, to obtain a seed culture broth. 2 ml of the seed culture broth was inoculated a brown rice solid medium (5 g of germinated brown rice, 2 ml of desalted water) in a 50 ml polypropylene tube. An aqueous solution of amino acid 1 was prepared to a concentration of 10 mg/ml and then sterilized by filtration. After 48, 72, 96 and 144 hours of culture, respectively, the amino acid solution was added to the polypropylene tube in an amount of 0.5 ml each, and stationary culture was performed at 27°C for 192 hours. 10 ml of methanol was added to the obtained culture broth, and the mixture was stirred by shaking at 180 rpm for 20 minutes, and then centrifuged at 3000 rpm for 10 minutes. 500 µl of the obtained methanol extract was concentrated and dried, and then dissolved in 10 µl of dimethyl sulfoxide and 90 µl of methanol. The solution was analyzed under the analysis condition indicated below as analysis condition 2, and as a result, production of compound 2 was observed. In the same manner, compound 2 was obtained by addition of amino acid 2, compound 3 was obtained by addition of amino acid 3, and compound 4 was obtained by addition of amino acid 4. The type of the amino acids added and the physicochemical properties of the compounds produced are shown in Table 3. With respect to the produced compounds, the specimens of compound 2, compound 3 and compound 4 were analyzed under the conditions of LC/MS shown as the analysis condition 2 to identify them on the basis that the M+H value and RT of mass spectrum coincided with the corresponding values of the produced compounds.

Analysis condition 2
Apparatus: LC-MS Agilent 1100 series ChemStations system, LCQ Navigator system
Column: Develosil Combi RP-5 (5 µm, 4.6 mm × 50 mm) (manufactured by Nomura Chemical Co., Ltd.)
Mobile phase: Solvent gradient elution shown below
Liquid A: water (0.01% trifluoroacetic acid)
Liquid B: acetonitrile (0.01% trifluoroacetic acid)

| Minute | % Liquid B |
|---|---|
| 0 | 15 |
| 0.7 | 15 |
| 1 | 35 |
| 5 | 55 |
| 6 | 82 |
| 7.2 | 98 |
| 7.6 | 98 |
| 8.2 | 15 |
| 8.8 | 15 |

Flow rate: 3 ml/min, Temperature: room temperature

**Table 3: Physicochemical properties of the compounds produced by amino acid-added culture**

| Amino acid added | Compound produced | RT (min) | ESI positive mode (M+H) | Molecular weight |
|---|---|---|---|---|
| Amino acid 1 | Compound 2 | 4.3 ± 0.1 | 653 | 652 |
| Amino acid 2 | Compound 2 | 4.4 ± 0.1 | 653 | 652 |
| Amino acid 3 | Compound 3 | 6.1 ± 0.1 | 670 | 669 |
| Amino acid 4 | Compound 4 | 5.8 ± 0.1 | 644 | 643 |

### Example 4

### Acquisition of strain F1476-G81

A spore suspension (number of spores: about 2x10⁶/ml) of *Fusarium* sp. strain F1476-C193 was prepared in the same manner as in Example 2, and 0.3 ml of the obtained spore suspension was subjected to a mutation treatment. 2 ml of the treated suspension was centrifuged at 14,000 rpm for 10 minutes, the supernatant was discarded, and the spores were washed with 2 ml of saline. The obtained spores were then diluted and applied to potato dextrose agar plates (manufactured by Nihon Pharmaceutical Co., Ltd.) in an amount of 0.1 ml each, cultured at room temperature for 5 to 6 days, and the resulting colonies were spread over a potato dextrose agar slant medium, to obtain 360 candidate mutant strains. The obtained strains were each inoculated to 10 ml of liquid medium 1 in a 30 ml polypropylene tube, and cultured with shaking at 160 rpm at 27°C for 7 days. 10 ml of n-butanol was added to the obtained culture broth, and the mixture was stirred by shaking at 180 rpm for 20 minutes, and then centrifuged at 3000 rpm for 10 minutes, to obtain a n-butanol extract. 1 ml of the obtained n-butanol extract was concentrated and dried, and then dissolved in 10 µl of dimethyl sulfoxide and 90 µl of methanol. The solution was analyzed under the analysis condition 3 described below, and high-producing strains for compound 1 were screened. As a result, a high-producing strain for compound 1, strain F1476-G81, could be obtained. Productivity of compound 1 of the strain F1476-G81 is shown as the concentration in n-butanol extract. The amount of production was quantified based on the peak area value obtained from chromatogram of absorption at 225 nm, by analyzing a standard product of the compound 1 under analysis condition 3 (Table 4).

**Table 4: Productivity for compound 1 of strain F1476-C193 and strain F1476-G81**

| Strain | Compound produced | Concentration (mg/L) |
|---|---|---|
| F1476-C193 | Compound 1 | <1 |
| F1476-G81 | Compound 1 | 1-10 |

RT for compound 1 under analysis condition 3: 2.0 ± 0.1 min

Analysis condition 3
Apparatus: Waters Millennium system 996
Column: Xterra MS C18 (5 µm, 4.6 mm × 250 mm) (manufactured by Waters Corporation)
Mobile phase: solvent gradient elution shown below
Liquid A: water (0.01% trifluoroacetic acid)
Liquid B: acetonitrile (0.01% trifluoroacetic acid)

| Minute | % Liquid B |
|---|---|
| 0 | 65 |
| 0.7 | 65 |
| 1 | 75 |
| 2.4 | 85 |
| 2.5 | 98 |
| 3.1 | 98 |
| 3.2 | 65 |
| 5 | 65 |

Flow rate: 1.5 ml/min, Temperature: room temperature

### Example 5

### Production of compound 4 by strain F1476-G81 by liquid culture method

A seed culture broth of strain F1476-G81 was prepared using liquid medium 1 in the same manner as in Example 2. 4 ml of the seed culture broth was inoculated into a 300 ml flask charged with 36 ml of liquid medium 2 (8.0% potato starch, 0.14% fructose, 0.8% bactosoytone, 2.0% rice bran, and 0.1% magnesium sulfate heptahydrate), and cultured with shaking at 220 rpm at 27°C. An aqueous solution of amino acid 5 was prepared to a concentration of 4 mg/ml and then sterilized by filtration. The amino acid solution was added to the flask in an amount of 0.5 ml each after 72, 96 and 144 hours of cultivation, respectively, and the culture with shaking at 220rpm was continued at 27°C for 168 hours. 4 ml of 100% ethanol was added to 1 ml of the culture broth, and the mixture was stirred by shaking at 180 rpm for 30 minutes, and then centrifuged at 3000 rpm for 10 minutes to obtain an ethanol extract. The obtained ethanol extract was analyzed under analysis condition 4 described below, and as a result, production of compound 4 was observed. The amount of production is shown as the concentration in the culture broth. The amount of production was quantified based on the peak area value obtained from chromatogram of absorption at 225 nm, by analyzing a standard product of the compound 4 under analysis condition 4 (Table 5).

**Table 5: Productivity for compound 4 in culture added with amino acid**

| Strain | Amino acid added | Amount of production of compound 4 (mg/L) |
|---|---|---|
| F1476-G81 | No addition | 0 |
| F1476-G81 | Amino acid 5 | 43 |

Physicochemical properties of compound 4
Molecular weight: 643
ESI (LC/MS positive mode) m/z = 644 (M+H)
RT under analysis condition 4: 1.3 ± 0.1 min

Analysis condition 4
Apparatus: LC-MS Agilent 1100 series ChemStations system Column: Xterra MS C18 (5 µm, 4.6 mm × 50 mm) (manufactured by Waters Corporation)
Mobile phase: solvent gradient elution shown below
Liquid A: water (0.01% trifluroacetic acid)
Liquid B: acetonitrile (0.01% trifluoroacetic acid)

| Minute | % Liquid B |
|---|---|
| 0 | 65 |
| 0.7 | 65 |
| 1 | 75 |
| 2.4 | 85 |
| 2.5 | 98 |
| 3.1 | 98 |
| 3.2 | 65 |
| 5 | 65 |

### Example 6

### Acquisition of strain F1476-H36

A spore suspension (number of spores: about 6x10⁷/ml) of *Fusarium* sp. strain F1476-G81 was prepared in the same manner as in Example 2, and 0..3 ml of the obtained spore suspension was subjected to a mutation treatment in the same manner as in Example 2. 2 ml of the treated suspension was centrifuged at 14,000 rpm for 10 minutes, the supernatant was discarded, and the spores were washed with 2 ml of sterilized saline. The spores were then diluted and applied to potato dextrose agar plates (manufactured by Nihon Pharmaceutical Co., Ltd.) in an amount of 0.1 ml each. The spores were cultured at room temperature for 5 to 6 days, and the resulting colonies were spread over a potato dextrose agar slant medium, to obtain 77 candidate mutant strains. The obtained mutants were each inoculated to 10 ml of liquid medium 1 in a 30 ml polypropylene tube, cultured with shaking at 160 rpm at 27°C for 3 days, to prepare a seed culture broth. 4 ml of the seed culture broth was inoculated to 36 ml of liquid medium 2 in a 300 ml flask, cultured with shaking at 220 rpm at 27°C for 7 days. 4 ml of 100% ethanol was added to 1 ml of the obtained culture broth, and the mixture was stirred by shaking at 180 rpm for 30 minutes, and then centrifuged at 3000 rpm for 10 minutes to obtain an ethanol extract. The obtained ethanol extract was analyzed under the analysis condition 3 described above, and high-producing strains for compound 1 were screened. As a result, a high-producing strain for compound 1, *Fusarium* sp. strain F1476-H36, could be obtained. The amount of production is shown as the concentration in the culture broth. The amount of production was quantified based on the peak area value obtained from chromatogram of absorption at 225 nm, by analyzing a standard product of the compound 1 under analysis condition 3 (Table 6).

**Table 6: Productivity for compound 1 of strain F1476-G81 and strain F1476-H36**

| Strain | Compound produced | Concentration (mg/L) |
|---|---|---|
| F1476-G81 | Compound 1 | 173 |
| F1476-H36 | Compound 1 | 588 |

### Example 7

### Production of compound 5 by strain F1476-H36 by liquid culture method

A seed culture broth of strain F1476-H36 was prepared using liquid medium 1 in the same manner as in Example 2. 4 ml of the seed culture broth was inoculated into a 300 ml flask containing 36 ml of liquid medium 3 (14.0% lactose, 0.1% glucose, 1.0% yeast extract, 2.0% Pharmamedia (manufactured by Traders Protein, Ltd.), and 0.1% magnesium sulfate heptahydrate), and cultured with shaking at 220 rpm at 27°C. An aqueous solution of amino acid 6 was prepared to a concentration of 4 mg/ml and then sterilized by filtration. After 72, 96 and 144 hours of cultivation, respectively, the amino acid solution was added to the flask in an amount of 0.5 ml each, and the culture with shaking at 220rpm was continued at 27°C for 168 hours. 4 ml of 100% ethanol was added to 1 ml of the obtained culture broth, and the mixture was stirred with shaking at 180 rpm for 30 minutes, and then centrifuged at 3000 rpm for 10 minutes to obtain an ethanol extract. The obtained ethanol extract was analyzed under analysis condition 4 described above, and as a result, production of compound 5 was observed. The amount of production is shown as the concentration in the culture broth. The amount of production was quantified based on the peak area value obtained from chromatogram of absorption at 225 nm, by analyzing a standard product of the compound 5 under analysis condition 4 (Table 7).

**Table 7: Productivity for compound 5 in culture added with amino acid**

| Strain | Amino acid added | Amount of production of compound 5 (mg/L) |
|---|---|---|
| F1476-G81 | No addition | 0 |
| F1476-G81 | Amino acid 6 | 71 |

Physicochemical properties of compound 5
Molecular weight: 681
ESI (LC/MS positive mode) m/z = 682 (M+H)
RT under analysis condition 4: 1.8 ± 0.1 min

### Example 8

### Production of compound 4 by strain F1476-H36 by liquid culture method

One loopful of microorganisms obtained from a slant culture of strain F1476-H36 was inoculated into a 500 ml Erlenmeyer flask with baffles containing 100 mL of liquid medium 4 (2.0% glucose, 1.5% glycerol, 1.0% potato starch, 0.25% polypeptone, 0.35% yeast extract, 0.5% calcium carbonate, 0.3% sodium chloride, 0.05% zinc sulfate heptahydrate, and 1.0% toasted soya bean), and culutured with shaking at 220 rpm at 27°C for 2 days, to obtain a seed culture broth. 4 ml of the seed culture broth was inoculated to 36 ml of liquid medium 5 (8.0% lactose, 0.1% glucose, 1.0% yeast extract, 1.0% Pharmamedia (manufactured by Traders Protein, Ltd.), and 0.1% magnesium sulfate heptahydrate) in a 300 ml flask, and cultured with shaking at 220 rpm at 27°C. An aqueous solution of amino acid 5 was prepared to a concentration of 6 mg/ml and then sterilized by filtration. After 72, 96 and 144 hours of culture, respectively, the amino acid solution was added to flask in an amount of 1 ml each, and the culture with shaking at 220rpm was continued at 27°C for 168 hours. 4 ml of 100% ethanol was added to 1 ml of the obtained culture broth, and the mixture was stirred by shaking at 180 rpm for 30 minutes, and then centrifuged at 3000 rpm for 10 minutes to obtain an ethanol extract. The obtained ethanol extract was analyzed under the analysis condition 4 described above, and as a result, production of compound 4 was observed. The amount of production is shown as the concentration in the culture broth. The amount of production was quantified based on the peak area value obtained from chromatogram of absorption at 225 nm, by analyzing a standard product of the compound 4 under analysis condition 4 (Table 8).

**Table 8: Productivity for compound 4 in culture added with amino acid**

| Strain | Amino acid added | Amount of production of compound 4 (mg/L) |
|---|---|---|
| F1476-H36 | No addition | 0 |
| F1476-H36 | Amino acid 5 | 253 |

### Example 9

### Acquisition of strain F1476-CH44.9

One loopful of microorganisms obtained from a slant culture of strain F1476-G81 was inoculated into a 250 ml Erlenmeyer flask charged with 30 ml of liquid medium 6 (4.0% fructose, 0.1% casitone, 0.3% sodium nitrate, 0.05% magnesium sulfate heptahydrate, 0.05% potassium chloride, and 0.001% iron sulfate heptahydrate), and cultured with shaking at 220 rpm at 27°C for 5 days. The obtained culture broth was centrifuged at 3000 rpm for 10 minutes, and a spore solution was prepared. Then, the obtained spore solution was subjected to a mutation treatment in the same manner as in Example 2. The treated solution was centrifuged at 14,000 rpm for 10 minutes, the supernatant was discarded, and the spores were washed with saline. The obtained spores were then diluted and applied to potato dextrose agar plates (manufactured by Nihon Pharmaceutical Co., Ltd.) in an amount of 0.1 ml each. The spores were cultured at room temperature for 5 to 6 days, to obtain candidate mutant strains. The obtained strains were inoculated to 30 ml of liquid medium 4 in a 250 ml Erlenmeyer flask, and cultured with shaking at 220 rpm at 27°C for 2 days, to obtain a seed culture broth. 4 ml of the seed culture broth was inoculated to 30 ml of liquid medium 5 in a 250 ml Erlenmeyer flask with baffles, and cultured with shaking at 220 rpm at 27°C for 7 days. 4 ml of 100% ethanol was added to 1 ml of the obtained culture broth, and the mixture was stirred by shaking at 180 rpm for 30 minutes, and then centrifuged at 3000 rpm for 10 minutes to obtain an ethanol extract. The obtained ethanol extract was analyzed under the analysis condition 3 described above, and strains not producing compound 1 were screened. As a result, a strain not producing compound 1, *Fusarium* sp. strain F1476-CH44.9, could be obtained.

### Example 10

### Production of compound 4 by strain F1476-CH44.9 by liquid culture method

One loopful of microorganisms obtained from a slant culture of strain F1476-CH44.9 was inoculated into a 500 ml Erlenmeyer flask with baffles containing 100 ml of liquid medium 4 (2.0% glucose, 1.5% glycerol, 1.0% potato starch, 0.25% polypeptone, 0.35% yeast extract, 0.5% calcium carbonate, 0.3% sodium chloride, 0.05% zinc sulfate heptahydrate, and 1.0% toasted soya bean), and cultured with shaking culture at 220 rpm at 27°C for 2 days, to obtain a seed culture broth. 4 ml of the obtained seed culture broth was inoculated to 36 ml of liquid medium 5 (8.0% lactose, 0.1% glucose, 1.0% yeast extract, 1.0% Pharmamedia (manufactured by Traders Protein, Ltd.), and 0.1% magnesium sulfate heptahydrate) in a 300 ml flask, and cultured with shaking at 220 rpm at 27°C. An aqueous solution of amino acid 5 was prepared to a concentration of 6 mg/ml and then sterilized by filtration. After 72, 96 and 144 hours of culture, respectively, the amino acid solution was added to flask in an amount of 1 ml each, and the culture with shaking at 220 rpm was continued at 27°C for 168 hours. 4 ml of 100% ethanol was added to the 1 ml of the obtained culture broth, and the mixture was stirred by shaking at 180 rpm for 30 minutes, and then centrifuged at 3000 rpm for 10 minutes to obtain an ethanol extract. The obtained ethanol extract was analyzed under the analysis condition 4 described above, and as a result, production of compound 4 was observed. The amount of production is shown as the concentration in the culture broth. The amount of production was quantified based on the peak area value obtained from chromatogram of absorption at 225 nm, by analyzing a standard product of the compound 4 under analysis condition 4 (Table 9).

**Table 9: Productivity for compound 4 in culture added with amino acid**

| Strain | Amino acid added | Amount of production of compound 4 (mg/L) |
|---|---|---|
| F1476-CH44.9 | No addition | 0 |
| F1476-CH44.9 | Amino acid 5 | 444 |

### Example 11

### Acquisition of strain F1476-CH44.28

One loopful of microorganisms obtained from a slant culture of strain F1476-CH44.9 were inoculated into a 250-ml Erlenmeyer flask containing 30 ml of liquid medium 6, and cultured with shaking at 220 rpm at 27°C for 5 days, to obtain a culture broth. The obtained culture broth was centrifuged at 3000 rpm for 10 minutes, and a spore solution was prepared from the supernatant. Then, the obtained spore solution was subjected to a mutation treatment in the same manner as in Example 2. The treated solution was centrifuged at 14,000 rpm for 10 minutes, the supernatant was discarded, and the spores were washed with saline. The spores were then diluted and applied to potato dextrose agar plates (manufactured by Nihon Pharmaceutical Co., Ltd.) in an amount of 0.1 ml each. The spores were cultured at room temperature for 5 to 6 days, to obtain candidate mutant strains. The obtained strains were inoculated into a 250 ml Erlenmeyer flask with baffles, containing 30 ml of liquid medium 4, and cultured with shaking culture at 220 rpm at 27°C for 2 days, to obtain a seed culture broth. 4 ml of the seed culture broth was inoculated into a 250 ml Erlenmeyer flask with baffles containing 30 ml of liquid medium 5, and a shaking culture was performed at 220 rpm at 27°C. An aqueous solution of amino acid 5 was prepared to a concentration of 6 mg/ml and then sterilized by filtration. After 72, 96 and 144 hours of culture, respectively, the amino acid solution was added to the flask in an amount of 1 ml each, and the culture with shaking at 220rpm was continued at 27°C for 168 hours. 4 ml of 100% ethanol was added to 1 ml of obtained culture broth, and the mixture was stirred by shaking at 180 rpm for 30 minutes, and then centrifuged at 3000 rpm for 10 minutes to obtain an ethanol extract. The obtained ethanol extract was analyzed under the analysis condition 3 described above, and high-producing strains for compound 4 were screened. By repeating the same mutation treatment, a high-producing strain for compound 4, *Fusarium* sp. strain F1476-CH44.28, could be obtained. The amount of production is shown as the concentration in the culture broth. The amount of production was quantified based on the peak area value obtained from chromatogram of absorption at 225 nm, by analyzing a standard product of the compound 4 under analysis condition 4 (Table 10).

**Table 10: Productivity for compound 4 in culture added with amino acid**

| Strain | Amino acid added | Amount of production of compound 4 (mg/L) |
|---|---|---|
| F1476-CH44.28 | No addition | 0 |
| F1476-CH44.28 | Amino acid 5 | 871 |

Structural formulas of amino acids and compounds

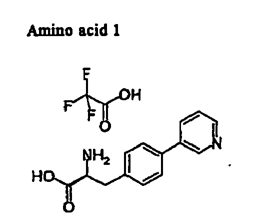

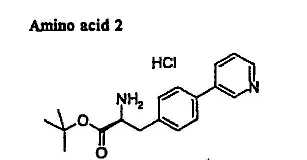

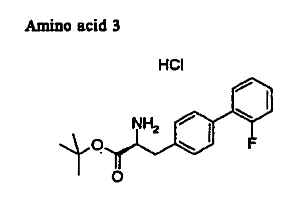

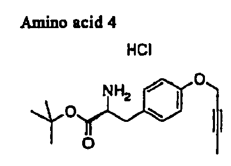

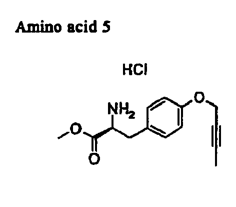

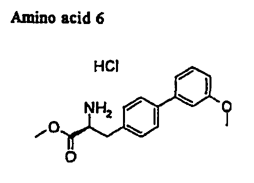

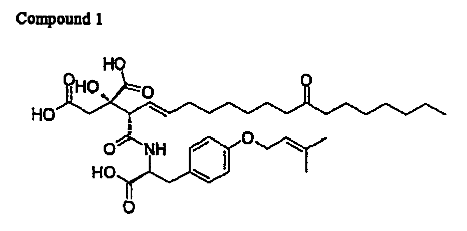

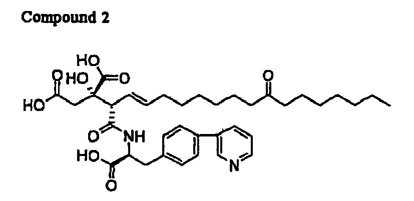

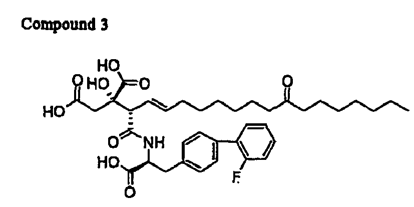

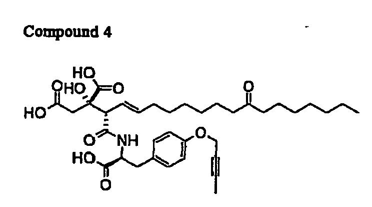

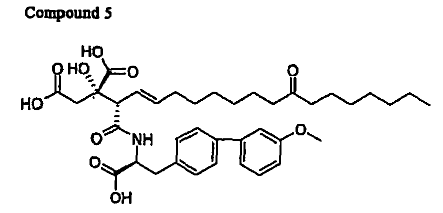

Hereinafter, examples of production methods of amino acid derivatives or salts thereof used in Examples described above will be described. Amino acid derivatives or salts thereof not described in the following are also commercially available, or can be easily synthesized on the basis of the description of the following Preparation Examples and technical knowledge of those skilled in the art.

### Preparation Example 1

Synthesis of (S)-2-amino-3-(4-pyridin-3-ylphenyl)-propionic acid trifluoroacetate (amino acid 1) 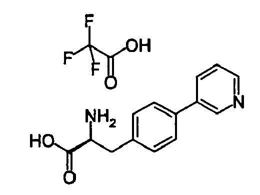 (S)-2-Amino-3-(4-pyridin-3-yl-phenyl)-propionic acid t-butyl ester (210 mg, 0.704 mmol) was dissolved in trifluoroacetic acid (1 ml), and the mixture was stirred at room temperature for 3 hours. After the solvent was removed under reduced pressure, a mixture (20 ml) of hexane-dichloromethane-methanol (10:3:1) was added to the obtained residue, then a precipitated powder was collected by filtration. After the powder was dried under reduced pressure by a vacuum pump, a colorless powder of (S)-2-amino-3-(4-pyridin-3-yl-phenyl)-propionic acid trifluoroacetate (245 mg, 98%) was obtained. Physicochemical properties of (S)-2-amino-3-(4-pyridin-3-ylphenyl)-propionic acid trifluoroacetate
Molecular weight: 356
EI-MS (positive mode): 242 (M⁺- C₂HF₃O₂)
¹H-NMR (D₂O) δ:
3.16(1H, dd, J=14.5Hz, 6.5Hz), 3.26(1H, dd, J=14.5Hz, 6.5Hz), 4.18(1H, t, J=6.5Hz), 7.38(2H, d, J=7.5Hz), 7.63(2H, d, J=7.5Hz), 7.99(1H, dd, J=8.0Hz, 6.5Hz), 8.60(1H, d, J=6.5Hz), 8.71(1H, d, J=8.0Hz), 8.91(1H, s)

### Preparation Example 2

Synthesis of (S)-2-amino-3-{4-(2-butynyloxy) phenyl}-propionic acid methyl ester hydrochloride (amino acid 5) 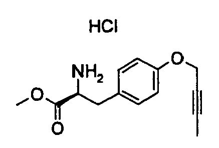 Potassium carbonate (87.7 g, 0.635 mol) and 1-bromo-2-butyne (74.3 g, 0.559 mol) were added to a solution of N-(t-butoxycarbonyl)-L-tyrosine methyl ester (Tokyo Chemical Industry Co., Ltd.; 150 g, 0.508 mol)in dry dimethylformamide(700 ml), and the mixture was stirred at room temperature for 19 hours. Ethyl acetate (2 L) was added to the reaction solution, and the mixture was washed sequentially with a saturated aqueous solution of ammonium chloride (1.5 L), a saturated aqueous solution of sodium hydrogencarbonate (1.5 L) and saturated saline (1.0 L). The ethyl acetate layer was dehydrated and dried over anhydrous sodium sulfate, then the solvent was removed under reduced pressure to yield light yellow oil. The obtained oil was dissolved in ethyl acetate (500 ml), and the solution was cooled to 0°C to 5°C. Subsequently, to the mixture was added 4N hydrochloric acid/ethyl acetate (1.0 L, 4.0 mol) dropwise, and the mixture was stirred for 2 hours at the same temperature, and then stirred for 15 hours at room temperature. The reaction mixture was diluted with ethyl acetate (500 ml), and the precipitated powder was collected by filtration and washed with a mixture (1.0 L) of hexane-ethyl acetate (2:1). After the washed powder was dried under reduced pressure by a vacuum pump, a colorless powder of (S)-2-amino-3-{4-(2-butynyloxy)phenyl}-propionic acid methyl ester hydrochloride (143 g, 99%) was obtained.
Physicochemical properties of (S)-2-amino-3-{4-(2-butynyloxy)phenyl)-propionic acid methyl ester hydrochloride
Molecular weight: 283
ESI (LC/MS positive mode) 248 (M-HCl+H⁺)
¹H-NMR (CD₃OD) δ :
1.81(3H, t, J=2.5Hz), 3.11 (1H, dd, J=14.5Hz, 7.0Hz), 3.21(1H, dd, J=14.5Hz, 6.0Hz), 3.81(3H, s), 4.27(1H, dd, J=7.0Hz, 6.0Hz), 4.66(2H, q, J=2.5Hz), 6.96(2H, d, J=8.5Hz), 7.17(2H, d, J=8.5Hz)

### Preparation Example 3

Synthesis of (S)-2-amino-3-(3'-methoxybiphenyl-4-yl)-propionic acid methyl ester hydrochloride (amino acid 6)

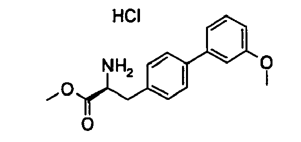

a) Synthesis of (S)-2-tert-butoxycarbonylamino-3-(4-trifluoromethanesulfonyloxyphenyl)-propionic acid methyl ester

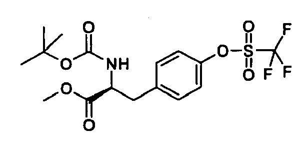

To a solution of N-(t-butoxycarbonyl)-L-tyrosine methyl ester (150 g, 0.508 mol)in dry dichloromethane (400 ml), dry pyridine (257 ml, 2.54 mol) was added, and the mixture was cooled to 0°C to 5°C. Subsequently, trifluoromethanesulfonic anhydride (143 g, 0.508 mol) was added dropwise, and the mixture was stirred for 2 hours and 30 minutes at the same temperature. Water (1.5 L) and dichloromethane (500 ml) were added to the reaction solution, the mixture was partitioned, and the organic layer was washed sequentially with a 0.5 N sodium hydroxide solution (1.5 L), water (1 L), 1N hydrochloric acid (2x1.5 L), and water (2x1.5 L). The organic layer was dried over anhydrous sodium sulfate, and concentrated to obtain (S)-2-t-butoxycarbonylamino-3-(4-trifluoromethanesulfonyloxyphenyl)-propionic acid methyl ester (217 g, 100%) as a milky white solid. Physicochemical properties of (S)-2-t-butoxycarbonylamino-3-(4-trifluoromethanesulfonyloxyphenyl)-propionic acid methyl ester
Molecular weight: 427
FAB-MS (positive mode, matrix m-NBA) 428 (M+H⁺)
¹H-NMR (CDCl₃) δ :
1.41(9H, s), 3.04(1H, dd, J=14.0Hz, 6.5Hz), 3.18(1H, dd, J=14.0Hz, 6.0Hz), 3.72(3H, s), 4.57-4.64(1H, m), 5.04(1H, brd, J=7.5Hz), 7.16-7.26(4H, m)

b) Synthesis of (S)-2-t-butoxycarbonylamino-3-(3'-methoxybiphenyl-4-yl)-propionic acid methyl ester 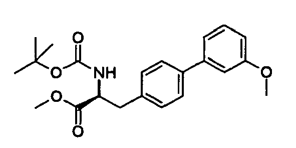
To a suspension of (S)-2-t-butoxycarbonylamino-3-(4-trifluoromethanesulfonyloxyphenyl)-propionic acid methyl ester (31.0 g, 0.0725 mol), 3-methoxyphenylboric acid (19.84 g, 0.131 mol), and potassium carbonate (14.03 g, 0.102 mol) in dry toluene (400 ml)was added tetrakis(triphenylphosphine) platinum (2.51 g, 0.0022 mol) under nitrogen atmosphere. The mixture was stirred for 2 hours at 90°C under nitrogen stream. The reaction mixture was filtered through Celite, and the residue was washed with ethyl acetate (500 ml). The filtrate was washed sequentially with a 0.5 N sodium hydroxide solution (2x500 ml), water (500 ml), 1N hydrochloric acid (500 ml), water (500 ml), and saturated saline (300 ml). The organic layer was dried over anhydrous sodium sulfate and concentrated, and then a crude compound (S)-2-t-butoxycarbonylamino-3-(3'-methoxybiphenyl-4-yl)-propionic acid methyl ester was obtained as a yellow oil. Physicochemical properties of (S)-2-t-butoxycarbonylamino-3-(3'-methoxybiphenyl-4-yl)-propionic acid methyl ester Molecular weight: 385
ESI (LC/MS positive mode) 386 (M+H⁺)
¹H-NMR (CDCl₃) δ:
1.42(9H, s), 3.00-3.20(2H, m), 3.74(3H, s), 3.86(3H, s), 4.59-4.67(1H, m), 5.02(1H, brd, J=8.0Hz), 6.87-6.92(1H, m), 7.10-7.32(4H, m), 7.38(1H, t, J=8.0Hz), 7.52(2H, d, J=8.5Hz)

c) Synthesis of (S)-2-amino-3-(3'-methoxybiphenyl-4-yl)-propionic acid methyl ester hydrochloride (amino acid 6) 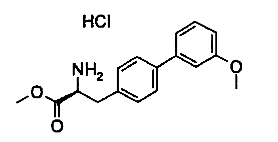
The oil obtained in b) was dissolved in ethyl acetate (100 ml), and the solution was cooled to 0°C to 5°C. Subsequently, to the solution was added 4N hydrochloric acid/ethyl acetate (83 ml, 0.332 mol) dropwise, and the mixture was stirred for 2 hours at the same temperature, and then stirred for 15 hours at room temperature. After the reaction mixture was diluted with hexane (100 ml), a precipitated powder was collected by filtration and washed with a mixture (50 ml) of hexane-ethyl acetate (2:1). After the washed powder was dried under reduced pressure by a vacuum pump, a colorless powder of (S)-2-amino-3-(3'-methoxybiphenyl-4-yl)-propionic acid methyl ester hydrochloride (20.63 g, 88%) was obtained.
Physicochemical properties of (S)-2-amino-3-(3'-methoxybiphenyl-4-yl)-propionic acid methyl ester hydrochloride
Molecular weight: 321
ESI (LC/MS positive mode) 286 (M-HCl+H⁺)
¹H-NMR (CD₃OD) δ:
3.21(1H, dd, J=14.0Hz, 7.5Hz), 3.32(1H, dd, J=14.0Hz, 6.5Hz), 3.84(6H, s), 4.37(1H, dd, J=7.5Hz, 6.5Hz), 6.92(1H, ddd, J=8.0Hz, 2.5Hz, 1.0Hz), 7.13(1H, dd, J=2.5Hz, 2.0Hz), 7.18(1H, ddd, J=7.5Hz, 2.0Hz, 1.0Hz), 7.33(2H, d, J=8.5Hz), 7.35(1H, dd, J=8.0Hz, 7.5Hz), 7.63(2H, d, J=8.5Hz)

### Test Example 1

### Identification of Fusarium sp. strain F1476

### Summary

Growth of the colonies of *Fusarium* sp. strain F1476 is very slow, and the strain is polymorphic as it forms light orange-colored wet sporodochia on a potato dextrose agar medium (PDA), or forms mycelia having fluffy, undulated and raised surfaces. However, the front and reverse sides never become reddish purple in color. From this finding, it was suggested that *Fusarium* sp. strain F1476 was *Fusarium merismoides, F. aquaeductuum*, or the like. *Fusarium* sp. strain F1476 formed plane and almost colorless colonies on a synthetic nutrient agar medium (SNA). The conidium is formed at the apex of a phialide which directly rises vertically from a sporodochium or hypha, or at the apex of a polyphialide which rises from an aerial hypha during late state of culture. The morphology is typically lunate in shape, with 0 to 5 septa and a length of 40 µm or less, and the apexes never spindle. The conidia have well-defined foot cells at the base, or has a abrupt shape or an obtuse shape. From these microscopic morphological features, the possibility for *Fusarium* sp. strain F1476 to belong to the Section Eupionnotes was denied, and thus *F. incarnatum* became a candidate.

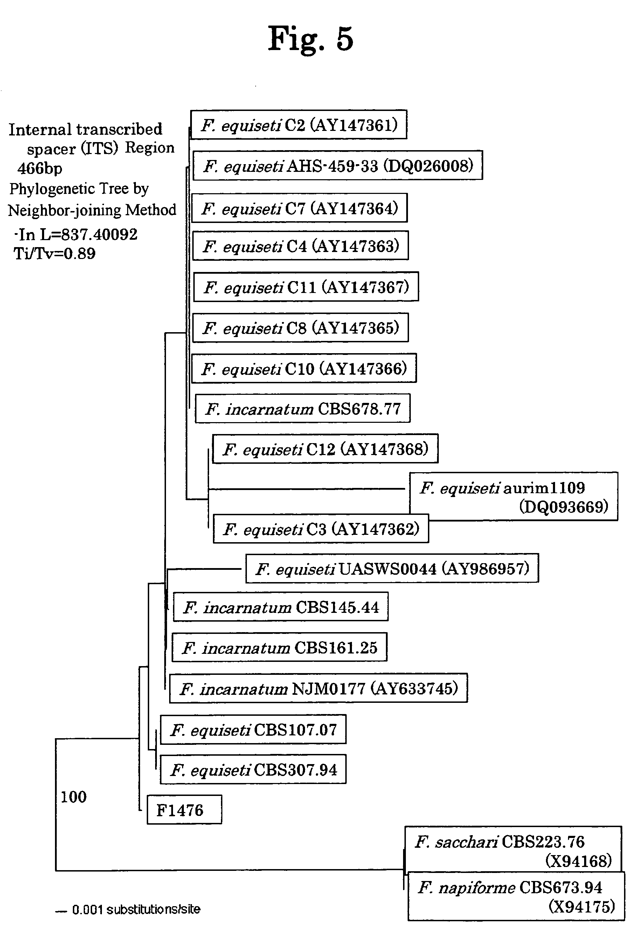

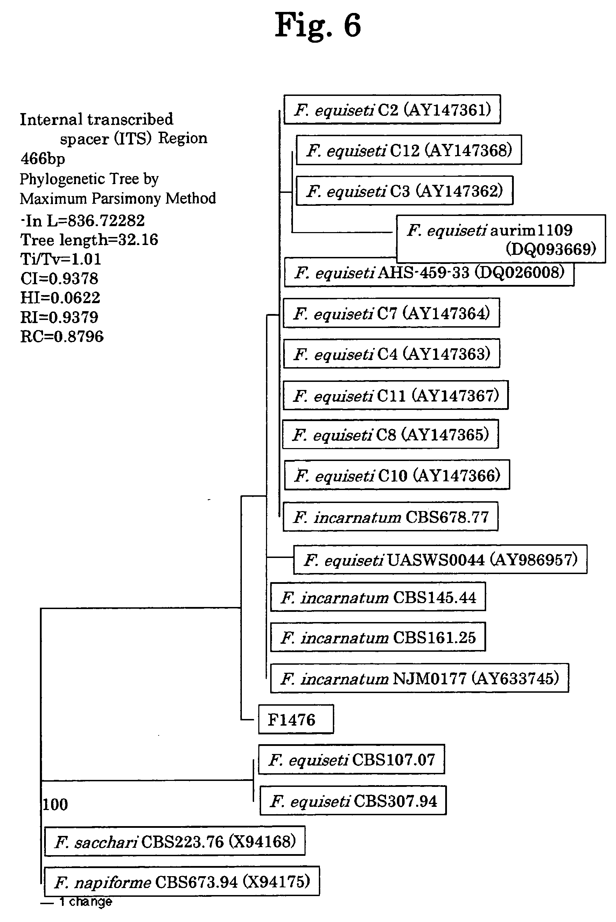

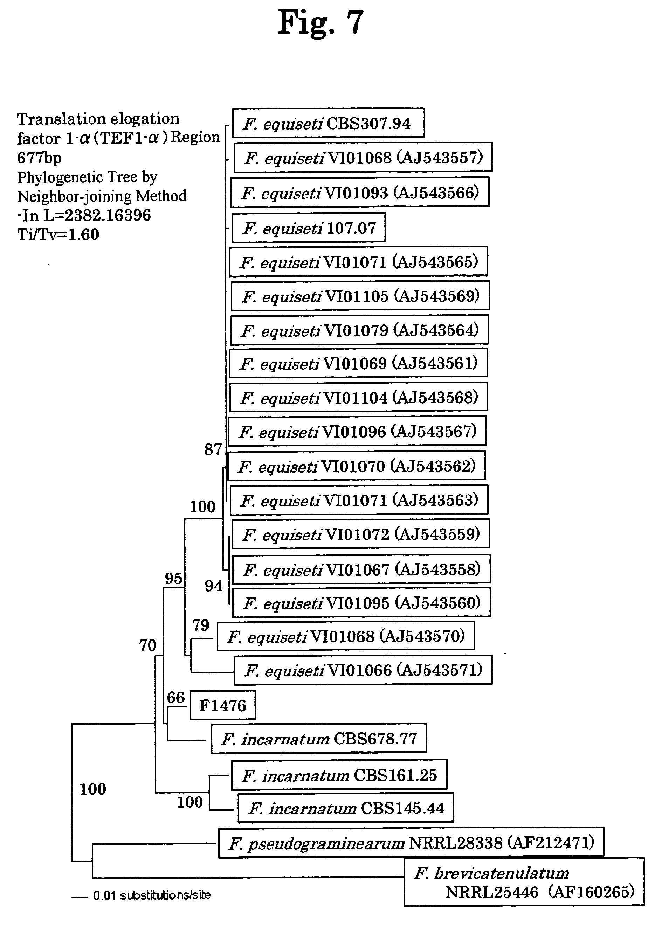

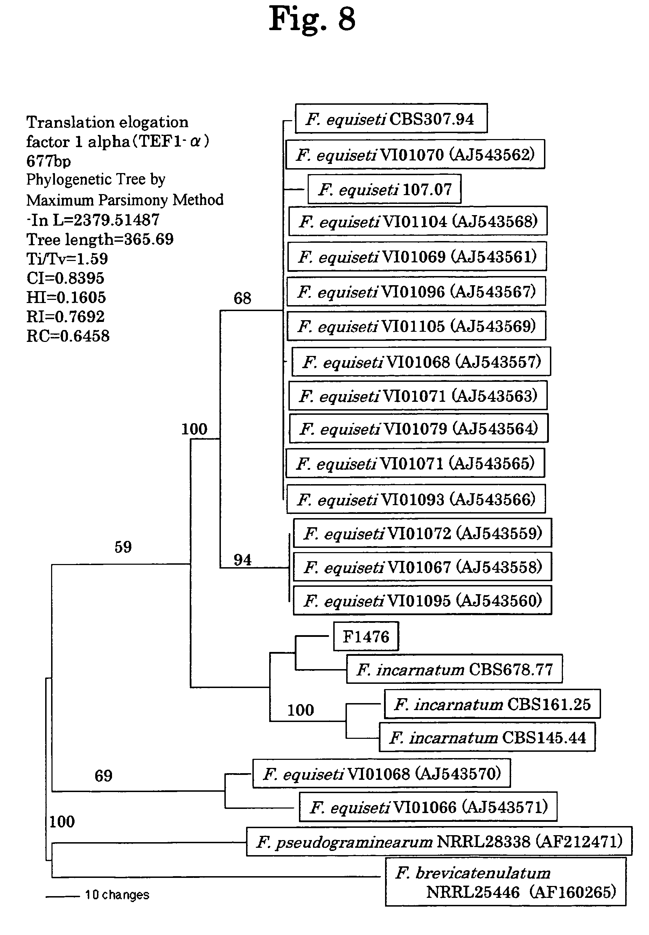

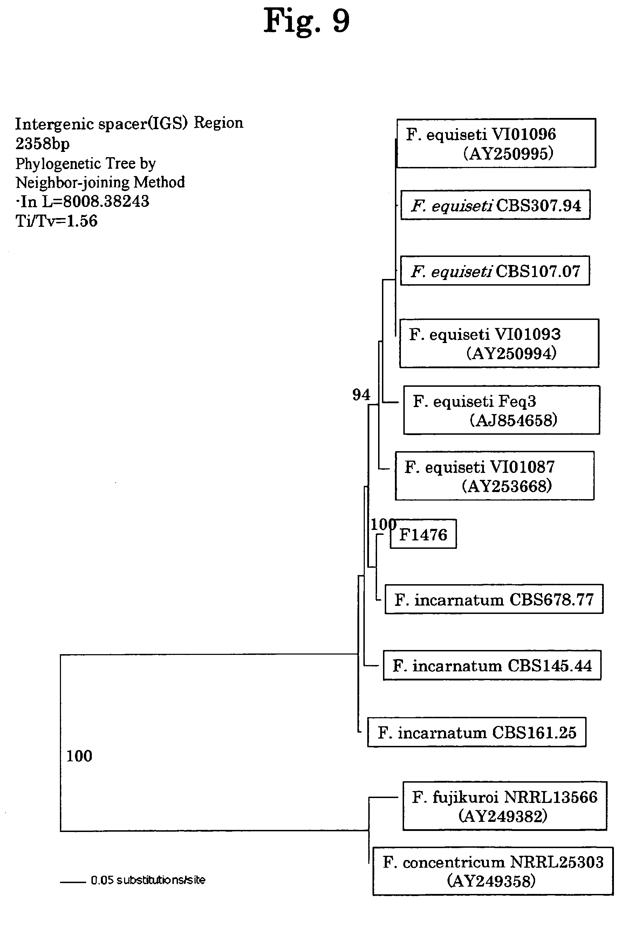

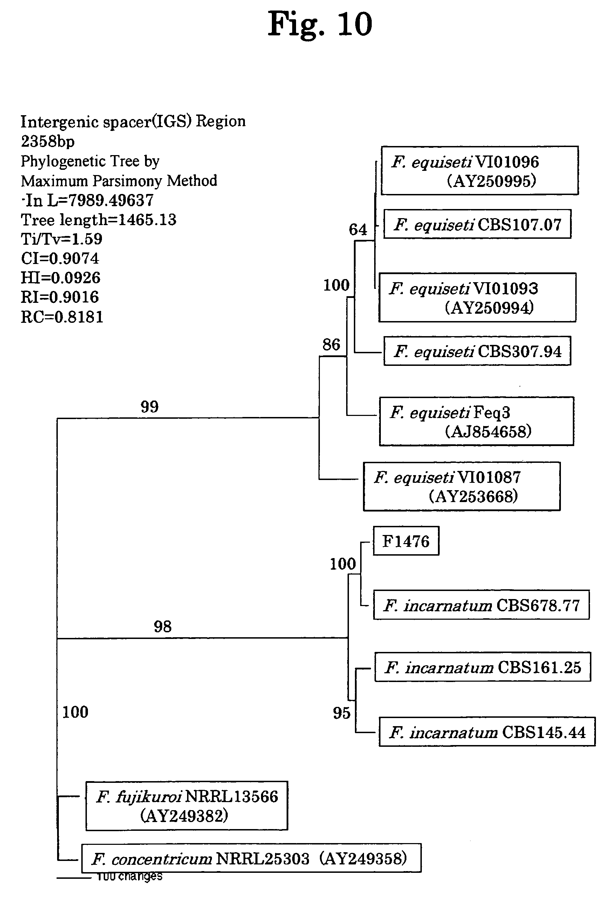

In order to perform a molecular phylogenetic analysis, the nucleotide sequences of the three regions of internal transcribed spacer (ITS) (Figs. 5 and 6), translation elongation factor 1 alpha (TEF 1-α) (Figs. 7 and 8) and intergenic spacer (IGS) (Figs. 9 and 10) were determined. Thus, it was found that strain F1476 was molecular phylogenetically closely related to *Fusarium equiseti* and *F. incarnatum,* and in particular, the strain was included in the clade of *F. incarnatum* and most closely related to *F. incarnatum* CBS 678.77. Meanwhile, since *F. equiseti* has a feature that the apexes of the conidia spindle, the possibility for strain F1476 to belong to *F. equiseti* was denied.

The above-described results were taken together, and the strain F1476 was identified as *Fusarium incarnatum* (Roberge) Saccardo. In addition, the validity of this identification was also proven from the fact that *F. incarnatum* CBS 678.77 produces the compound 1, as the strain F1476 does.

### Materials and Methods

### Fungal strain used

*Fusarium* sp. strain F1476 is a filamentous fungi separated from the fallen leaves collected at the southern slope of Kamakurayama in Kamakura city on January 24, 2000, by a washing and filtration method on February 29, 2000. As will be described later, the strain was prone to natural mutation, and clones having different properties for culture occur easily. In some cases, clones forming heterogeneous colonies are referred to as org, clones making orange-colored sporodochia all over the colonies surface are referred to as wet, and clones formed mainly of hyphae are referred to as fast.

The fungal strains used for the comparison are all strains provided from Centraalbureau voor Schimmelcultures, Utrecht (hereinafter, also referred to as "CBS"). *Fusarium equiseti* (Corda) Sacc. CBS 107.07, CBS 193.60, CBS 307.94, *Fusarium incarnatum* (Rob.) Sacc. CBS 161.25, CBS 145.44, CBS 678.77. The experiments of culturing the fungal strains provided from CBS were all performed by Professor Toru Okuda at Tamagawa University.

### Observation of morphological properties

The methods for morphology observation and the media used were mainly in accordance with Gerlach and Nirenberg (1982) and Aoki (1998 and 2003). Specifically, conidia or hypha fragments taken from a well grown slant were inoculated at three spots each on a potato dextrose agar medium (PDA) and a synthetic nutrient agar medium (SNA), and cultured in dark or under irradiation with near ultraviolet light, at 20°C or 25°C for 1 week to 3 weeks (mainly 10 days). The properties for culture were observed with naked eyes or with a stereomicroscope, and the colors of conidia or mycelia were described as Munsell and Methuen symbols. Furthermore, culture media such as wheat germ extract agar medium (MA), oatmeal agar medium (OA) and Miura medium (LCA) were also used. For the observation of morphology, PDA and SNA were predominantly used, and carnation leaf agar medium (CLA) was also occasionally used. For the measurement of phialide or conidia, Adobe Photoshop (ver 7.0, Adobe Systems, Inc.) and a general purpose image analytical software, Optimas (ver 6.5, Media Cybernetics, Inc.), were used to perform semi-automatic measurement.

### Extraction of DNA

Strain F1476, and *F. equiseti* and *F. incarnatum* supplied from CBS were cultured on PDA, and their DNA were extracted using a QIAamp DNA Mini Kit (Qiagen Corporation, Tokyo).

For the amplification of Internal transcribed spacer (ITS) region, ITS4 (TCCTCCGCTTATTGATATGC, SEQ ID No. 1) primer and ITS5 (GGAAGTAAAAGTCGTAACAAGG, SEQ ID No. 2) primer (White et al., 1990) were used; for the amplification of translation elongation factor 1 alpha (TEF 1-α) region, TEF1 (ATGGGTAAGGA(A/G)GACAAGAC, SEQ ID No. 3) primer and TEF2 (GGA(G/A)GTACCAGT(G/C)ATCATGTT, SEQ ID No. 4) primer (O'Donnell et al., 1998) were used; and for the amplification of intergenic spacer (IGS) region, CNL12 (CTGAACGCCTCTAAGTCAG, SEQ ID No. 5) primer and SCN1 (GAGACAAGCATATGACTACTG, SEQ ID No. 6) primer (Kosiak et al., 2005) were used. Amplified DNAs were purified using a QIAamp Purification Kit (Qiagen Corporation, Tokyo). The purified samples were subjected to cycle sequencing, using a Big Dye Terminator v3.1 Cycle Sequencing Kit (Applied Biosystems Japan Co., Ltd., Tokyo) and the above-mentioned 6 species of primers, and for the IGS region, using 8 species of primers in total, including newly designed IGS1 (TCACCAATCACTAACTTCCTCTTCCG, SEQ ID No. 7) primer and IGS2 (TGGGATCCTCAGCTTTTTCTGCAT, SEQ ID No. 8) primer. After purification, DNA sequenceing was performed using Genetic Analyzer (ABI 310, Applied Biosystems Japan Co., Ltd., Tokyo). The resulting sequences of the ITS region, TEF 1-α region and IGS region of strain F1476 are presented in the following.

Sequence of IGS region of strain F1476 (SEQ ID No. 9)

CCCCAACGACAGACTCTCGCAGTGCGAGGCGTGGTAGGGGTTCTCACCTTTGGAAAG
ACGGGTCGAGCAGGCTTCGGCCTGCCGATGCGTCTAAGTCGAGGGTGTTCAGGGTAGG
CAGGTCAGACTTGGTGGAATCGGGAATCGGTTCGAGACGGGTTGCGGTCGTGGATGA
GGTTGATTTAAAGTTGCGGAGGTGCGGGTCTGCAGGGTAGGCAGGAGTGCCAGAGTCG
AGTTGAGAGCCCCTCTGGGACGAATAGGTGATGGTATGGGCCGGCTGCAGGGTAGGCG
GGGATTGTCTTGGTCGAGCTGGTTGTCGTCCGGGTGGTGAAAGGCGATCGGGGTCACT
GCAGGGTAGGCAGAAATGGCTTGGTGGGATGCTTGGACGGAAGAGGCGCCGGTCTCCT
GCCCAGGTCGTGTCGTGGGAAACCGGTGCGGAATGGTCTGGCGGTCCGGTGGTCGTGA

GCACATTTTTAAAATCCGCCATACAAATGAATTTTGCGGAAAATCAAAGTTGGCCCGC
GAGACGGCTCGGGCGAGCGGCCGGCTGGAGAGCCCCGGAGGACCTATGAGAGAAACG
GCGCCGGGAGGGGCCCAAGGAGAGCTGCAGGGTAGGCAGCCCCAGGTTGGCGTGGAG
GCTCGGTGCTCTTGTCCTTTTGTTGCTAGCTTTCGATGTCCGCATTCACTCGGTCGTC
TGGTCGGTCGTCCGGCTGGTCAGGCAGAAAGTCACCCTGACACTCGTCAGTCACGAGC
TCATGCGTTCCGTGGGAGGTCACGTGATCGGTCACGTGCTTTTGTATATAGCTGATTT
AACTGATGGAAGTGTCTTAGTCAGCAAAAAATAACTAATTACAATATGATTATCGAGT
TGTTGACGGATTTTGCCTATGTGTAAATGCGGAGATGTTGAGAAAGGTACGGAAATT
CAAAACCACCAACCTTGTGACCGGAAACGCAGCTTTGCGTGTGACCATGGATGCAGAA
AAAGCTGACGATCCCAAAACACCTTTCCTTTTCCCCGTACGACGACGGTCGAGGCGTG
AGGTTGTTTTCGTGTCGATGTCGGACTGATTTAAGGTAGGCAGGATCAACTTCTTGCC
TACCCGGTAGCTGAGATTCAAAACGACCTGGCGCCCGCCACAGACCTCGCCCGAGGTG
GTGAGATATATCATTCGGATACGCCTTGGCCTGTTCTATGTGCCTAGGCACTCTTGTG
GAAACCAGTGCGAAATGGTTTTGCGGTTTGGTGGTCGTGAGCACATTTTTAAAATTTC
CCATACAAATGAATTTTGCGGAAAATAAAAAGTGGCCCACGAGCCCGATCTGGCGTGC
GGCCGACTAAAACATTCTCGGAGGGTATATGAGAGGGGAGCAAAGCAGCCCGGCCCGA
AAGGGTGTGGACAAAGCTGCGGCGCGAACCTCCGTACCTGATCTTGATAGCCGACCAC
TGTGGCTTCCCCGGCGTGCAGGCGTCTTTCGGACAAACCCCCGGAAGAGGAAGTTAGT
GATTGGTGACCCCCGTGAAGCGTCTGGAATAACACGCGTAGCGGGGACCAAGTCCTTC
AACCAGCCTGAGGCATACCACCGAGGCTGCTGGGGGCACTGGGCTCTGTGGATGGCTG
GCCACTGGCTAGTACTGAAACTTAGCACATCGGGGGAAACCACCCGCTGCTGAAGCCC
TCGCGGCAGACGCAGCGGGCGCGGTCCTCCACCATGGGCTCCTAAGCCACAGCACCCT
TCGGGGCTCGCTGCGGCGGACGGTAGCCCTGGAAAACCCAGAGTGGAAAGCGGAACG
CCTTGCGAGGCGCGACTGGCCCTGCCGGAAGCGCTGAGCTTGGTTCAGCCCGGCGAAA
GGTGTAAAAACCTCTAGCTGCTGTAACTAGTGAGCTCTCCGGCGCCTT

Sequence of TEF 1-α region of strain F1476 (SEQ ID No. 10)

GTCGACTCTGGCAAGTCGACCACTGTGAGTACTACCCTCAATGACCTTGCTTATCAGC
AGTCATCAACCCCCCATACGTGGCGGGTAATTTCAACTTGAATATTGGCTGACAAAA
TTGCATAGACCGGTCACTTGATCTACCAGTGCGGTGGTATCGACAAGCGAACCATCGA
GAAGTTCGAGAAGGTTGGTTTCCATTTTCCTCGATCGCACGCCCTCTGCCCACCGATC
AATCACCCGAATCCGTCTTACGACGACTGAATATGCGCCTGTTACCCCGCTCGAGTAC
AAAATTTTGCGGTTCAATCGTAATTTTTGGTGGGGCTTATACCCCGCTACTCGAGTGA
CAGGCGTTTGCCCTTTCCCACAAAATCATCTCTTGCGCATCACGTGTCAAACAGTCAC
TAACCACCCGACAATAGGAAGCCGCCGAGCTCGGTAAGGGTTCCTTCAAGTACGCCTG
GGTTCTTGACAAGCTCATAGGCCGAGCGTGAGCGTGGTATCACCATCGATATCGCCCT

CTGGAAGTTCGAGACTCCTCGCTACTATGTCACCGTCATTGGTATGTTGTCAATCGCT
TACACTCGTTACCTTCTTATGCCAAACATGTGTTCCAGACGCTCCCGGTCCACCGTA

Sequence of ITS region of strain F1476 (SEQ ID No. 11)

AGTTTACAACTCCCAAACCCCTGTGAACATACCTATACGTTGCCTCGGCGGATCAGCC
CGCGCCCCGTAAAACGGGACGGCCCGCCCGAGGACCCCTAAACTCTGTTTTTAGTGGA
ACTTCTGAGTAAAACAAACAAATAAATCAAAACTTTCAACAACGGATCTCTTGGTTCT
GGCATCGATGAAGAACGCAGCAAAATGCGATAAGTAATGTGAATTGCAGAATTCAGTG
AATCATCGAATCTTTGAACGCACATTGCGCCCGCCAGTATTCTGGCGGGCATGCCTGT
TCGAGCGTCATTTCAACCCTCAAGCCCAGCTTGGTGTTGGGACTCGCGGTAACCCGCG
TTCCCCAAATCGATTGGCGGTCACGTCGAGCTTCCATAGCGTAGTAATCATACACCTC
GTTACTGGTAATCGTCGCGGCCACGCCGTAAAACCCCAACTTCTGAATGTTGACC

### Phylogentic analysis

The obtained sequence data were aligned with Clustalw, and weighting was performed by determining the Ti/Tv ratio using PAUP (ver 4.0 beta10; Swofford 2000). Thereafter, the data were analyzed by a neighbor-joining method and a maximum parsimony method, a phylogenetic tree was prepared, and bootstrap calibration was performed.

### Confirmation of production of compound 1 by strain F1476 and CBS standard strain

Seven strains in total, including strain F1476 and standard strains of *F. equiseti* and *F. incarnatum,* were subjected to stationary culture in a buckwheat medium (10 g of buckwheat and 10 ml of SB solution are contained per one 250 ml Erlenmeyer flask. The SB solution consists of 100 mg of yeast extract (Oriental Yeast Co., Ltd.), 50 mg of sodium tartrate, 50 mg of KH₂PO₄, 50 mg of MgSO₄·7H₂O, 5.0 mg of FeSO₄·7PH₂O, 5.0 mg of ZnSO₄·7H₂O, and 1,000 ml of desalted water), in two plates for each strain, at 25°C for 12 days.

The cultured product was extracted with 20 ml of n-butanol, and 300 µl was concentrated and dried. The resultant concentrate was dissolved in an equivalent amount of methanol, to prepare a sample solution. Then, the sample solution was analyzed under the analysis conditions for LC/MS described in the following analysis condition 5.

Compound 1 thus detected was quantified by an absolute calibration curve method using the peak area value from mass chromatography (positive ion (M+H): 660), which value was obtained by analyzing a standard product under the LC/MS conditions described in the following analysis condition 5.

Analysis condition 5
Apparatus: Shimadzu high performance liquid chromatography mass spectrometer LCMS-2010A
Column: Shiseido C18 MGII, 2 mm × 75 mm, 3 µm (manufactured by Shiseido Co., Ltd.)
Mobile phase: solvent gradient elution shown below
Liquid A: water (0.1% formic acid)
Liquid B: acetonitrile (0.1% formic acid)

| Minute | % Liquid B |
|---|---|
| 0 | 20 |
| 1.0 | 20 |
| 20.0 | 80 |
| 26.0 | 80 |
| 27.0 | 20 |

Flow rate: 0.2 ml/min, Temperature: 40°C
Detection: Ionization; electrospray (ESI)
Scan mode: selective ion method (SIM) (positive, m/z = 660.3, 592.1)

Next, a sample solution obtained by culturing *F. incarnatum* CBS 678.77 was analyzed using the analysis conditions for TOF-MS described in the following analysis condition 6.

Analysis condition 6
Apparatus: LC-MS Agilent 1100 series ChemStations system, LCQ Navigator system
Column: Develosil Combi-RP, 3.0 × 50 mm, 5 µm (manufactured by Nomura Chemical Co., Ltd.)
Mobile phase: solvent gradient elution shown below
Liquid A: water (0.01% trifluoroacetic acid)
Liquid B: acetonitrile (0.01% trifluoroacetic acid)

| Minute | % Liquid B |
|---|---|
| 0 | 15 |
| 0.7 | 15 |
| 1.3 | 35 |
| 2.6 | 35 |
| 4.5 | 55 |
| 6 | 82 |
| 6.8 | 82 |
| 7.2 | 98 |
| 8 | 98 |
| 8.1 | 15 |
| 10 | 15 |

Flow rate: 1.276 ml/min, Temperature: 60°C

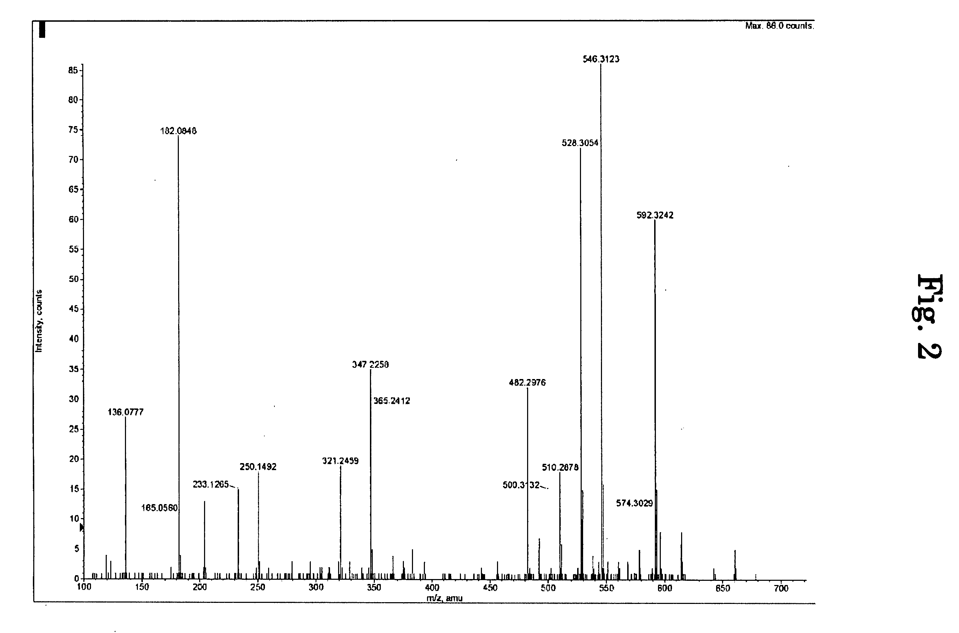

Likewise, a sample solution obtained by culturing *F. incarnatum* CBS 678.7 was subjected to an MS/MS analysis using the analysis conditions described in the following analysis condition 7, and the results were compared with the spectrum (FIG. 2) obtained from a standard product of compound 1.

Analysis condition 7
Apparatus, column, mobile phase, flow rate, temperature: the same as those used in analysis condition 6
MS/MS conditions
Ion mode: ESI positive
Ion spray voltage: 5500 V
Temperature: 450°C
Collision energy: 25 V

### Results

### Various properties for culture

Growth of *Fusarium* sp. strain F1476 on potato dextrose agar medium (PDA) was slow, the colony size reached a diameter of 10 to 15 mm after 10 days under irradiation with near-ultraviolet light at 25°C, and the growth rate was 1.5 to 1.6 mm/day. *Fusarium* sp. strain F1476 formed partially dense, rigid hyphae, which had undulated and raised surfaces exhibiting white to light pink color (Munsell 5YR-10YR9/2), with conspicuously fluffy masses of hyphae being occasionally formed on the periphery (referred to as fast part). Further, wet sporodochia were concentrated in some parts, and the color was light orange (apricot color, light orange, Light Apricot to Apricot, Munsell 5YR7/6 to 7/10, Methuen 6A6 to 6B8) (referred to as wet part). The reverse side exhibited a color ranging from light orange to orange (light orange, bright reddish orange, Tiger Lily, Munsell 5-10YR7/10, Methuen 6B8 to 8A6), but the front and reverse sides did not turn reddish purple. The growth rate was slightly poor in dark, being 0.9 to 1.1 mm/day. The color tone was lighter and beige (pale beige, Ivory, Munsell 10YR9/2, Methuen 4A3), while the reverse side color was light reddish yellow (light reddish yellow, Naples Yellow). However, the color tone of the hypha could occasionally become darker, ranging from ocher to grayish brown (gold, Golden Ocher, light grayish brown, Blond, Munsell 10YR5/4-8, Methuen 5E5-8). In this case, the reverse side color was dark yellowish brown (chestnut, dark yellowish brown, Burnt Umber, Munsell 10YR3/6). In any of the cases, the reverse side did not turn reddish purple.

When the part richly forming wet sporodochia (WET part) as described above was subcultured, growth became faster, reaching a diameter of 22 to 27 mm in 10 days, and homogeneous flat, velvet-like, mucous, orange colored colonies were formed. Sometimes, the strain produced a yellow dye in the medium. Also, when the FAST part was subcultured, the orange color of sporodochia was not recognized with naked eyes, and fluffy or granular or powdery colonies, mainly composed of hyphae, were formed, and the colonies reached 10 to 16 mm after 10 days.
Growth of *Fusarium* sp. strain F1476 on a synthetic nutrient agar medium (SNA) was slow, and the colony size reached a diameter of 13 mm after 7 days under irradiation with near-ultraviolet light, with the growth rate being 1.6 mm/day. *Fusarium* sp. strain F1476 formed flat and thin colonies. The colonies were felt or wooly texture, and had wet sporodochia sporadically present in the central part. The color tone of the colonies was beige (pale beige, Ivory, Munsell 10YR9/2, Methuen 4A3), and the reverse side of the colonies had the same color. Similar growth was observed in dark.

The WET part was subcultured and was grown on SNA. The colonies reached 14 to 19 mm after 10 days, but there were no significant changes in other properties for culture. The FAST part showed fast growth to reach 19 to 20 mm after 10 days.

Growth of *Fusarium* sp. strain F1476 on malt extract agar medium (MA) was slow, and the colony size reached a diameter of 11 mm after 11 days under irradiation with near-ultraviolet light, with the growth rate being 1.0 mm/day. *Fusarium* sp. strain F1476 formed dense hyphae, and the hyphae were raised and exhibited a wooly or felt texture. The color tone of the colonies was light orange (light orange, Light Apricot, Munsell 5YR8/6, Methuen 6A6), and of the reverse side had a bright orange color (bright orange, Nasturtium Orange, Munsell 5YR7/12, Methuen 6A7). The color tone of the colonies was paler in dark.

Growth of *Fusarium* sp. strain F1476 on oatmeal agar medium (OA) was fast, and the organism reached a diameter of 21 mm after 10 days under irradiation with near ultraviolet light, with the growth rate being 3.7 mm/day. *Fusarium* sp. strain F1476 presented granular colonies which were flat, but had a large number of sporodochia densely concentrated in the central part. The color tone of the colonies was light yellowish orange (light yellowish orange, Maize, Munsell 5YR7/8, Methuen 6B6), and the reverse side had the same color. The color tone of the colones was paler in dark.

Growth of *Fusarium* sp. strain F1476 on Miura medium (LCA) was fast, and the organism reached a diameter of 25 mm after 10 days under irradiation with near ultraviolet light, with the growth rate being 3.4 mm/day. *Fusarium* sp. strain F1476 formed flat and thin colonies, and the colonies had a granular or fluffy texture, and had wet sporodochia sporadically present in the central part. The color tone of the colonies was beige (pale beige, Ivory, Munsell 10YR9/2, Methuen 4A3), and the reverse side of the colonies had the same color. Similar growth was observed in dark.

### Morphological properties

On SNA, conidiophores occasionally rise vertically mainly from aerial hypha and branch out, and 2 to 8 phialides verticillated directly at the branches or the conidiophores. The conidium-forming structure formed from conidiophores or phialides occasionally showed a complicated structure forming sporodochia. Furthermore, in the areas peripheral of the colonies, phialides grew individually or verticillated directly on aerial hyphae, without forming conidiophores. There were also cases where conidia were formed from hyphae groveling on the surface of agar. In particular, when cultured for two weeks or longer, conidium-forming cells stretched or swelled during the process of conidium formation, and became polyphialides (or meso-conidia forming cells) which generated a plurality of conidium ontogeny parts from a single cell. The conidiophores were 10 to 30 µm in length. The phialides were cylindrical, pen-like or bottle-like, and usually had a prominent cup-like structure on the apex. The polyphialides (or meso-conidia forming cells) had 2 to 5 openings generated in the upper part, but the cup-like structure was not prominent. In the conidium-forming cells (phialides, polyphialides), the size of individual cells was (8.5) 10.5 - 24.5 (36.5) × 2.5 - 3.0 (3.5) µm (average 17.5 × 3.0 µm), with L/W being (2.75) 3.10 - 9.70 (16.25) (average 6.4), for the wet part; and (7.0) 9.5 - 25.0 (28.0) × 2.5 - 3.0 (3.5) µm (average 17.0 × 3.0 µm), with L/W being (2.78) 3.31 - 8.77 (10.0)(average 6.04), for the fast part. Particularly, in the densely verticillated conidium-forming cells which form sporodochia, the size was (6.0) 8.0 - 14.0 (19.0) ×2.5 - 3.5 µm (average 11.0 × 3.0 µm), with L/W being (2.00) 2.38 - 5.06 (7.56) (average 3.72), for the wet part; and (8.5) 9.0 - 12.5 (14.0) × (2.5) 3.0 - 3.5 µm (average 11.0 × 3.0 µm), with L/W being (2.5) 2.85 - 4.33 (5.0) (average 3.59), for the fast part. The conidia were formed in mucous clumps at the apexes of a phialide, and typically took a lunate form, while occasionally taking oblong shapes or cylindrical shapes. The apex does not spindle conspicuously. The conidia have well-defined foot cells at the base, or have abrupt shapes or obtuse shapes. Furthermore, the conidia typically have 2 to 4 septa, but occasionally had no septum, or had 1 or 5 septa. The size of the conidia in the wet part was (18.5) 23.5 - 29.0 (32.5) × (3.0) 4.0 - 5.5 (6.5) µm (average 26.5 × 5.0 µm), with L/W being 4.19 - 8.39 (average 5.52), for a triseptated conidium; 27.0 - 30.0 (32.5) × (3.0) 3.5 - 5.5 (6.0) µm (average 28.5 × 4.5 µm), with L/W being 4.65 - 8.88 (average 6.48), for a quadriseptated conidium; and in the fast part, the size was 16.0 - 19.0 × 2.5 - 3.0 µm (average 17.5 × 2.5 µm), with L/W being 6.45 - 6.84 (average 6.64), for a biseptated conidium; (15.5) 22.0 - 28.0 (33.5) × (2.5) 3.0 - 5.0 (5.5) µm (average 25.0 × 4.0 µm), with L/W being 4.42 - 10.30 (average 6.55), for a triseptated conidium; (25.0) 26.0 - 31.5 (37.0) × (3.0) 4.0 - 5.5 (7.0) µm (average 29.0 × 4.5 µm), with L/W being 4.45 - 9.41 (average 6.4), for a quadriseptated conidium; and 27.5 - 33.0 (35.5) ×(3.0) 4.0 - 5.5 (6.0) µm (average 30.5 ×5.0 µm), with L/W being 4.87 - 8.81 (average 6.54), for a pentaseptated conidium. Although formation of chlamydospores having typical rough surfaces was not observed on the hyphae, hollowed and thickened, nearly flat subspherical cells were formed in the form of individual cells or a chain of several cells, at the apexes or in the middle of the hyphae. The size was (5.5) 8.5 - 15.0 (15.5) ×(5.0) 6.0 - 13.5 (14.0) µm (average 11.5 ×9.5 µm), with L/W being 1.04 - 1.45 (1.61) (average 1.24) (see Fig. 3 and Fig. 4). In addition, for example, the expression "(5.5) 8.5 - 15.0 (15.5)" means that "the size is usually in the range of 8.5 to 15.0, but may be extended to 5.5 to 15.5."

### Phylogenetic analysis based on DNA sequences

The nucleotide sequence of the ITS region (466 bp) obtained was subjected to BLAST search, and as a result, the strain F1476 was found to have high homology with *F. equiseti* and *F. incarnatum.* For the phylogenetic analysis, registered data of the standard strains of strain F1476 and CBS, 3 strains of *F. equiseti,* and 3 strains of *F. incarnatum,* as well as those of 14 strains found by BLAST search to have high homology, were used. As a result of phylogenetically analyzing the data by a neighbor-joining method and a maximum parsimony method (Figs. 5 and 6), strain F1476 formed a clade with *F. equiseti* and *F. incarnatum* (bootstrap value: 100).

For the phylogenetic analysis of the TEF 1-α region (677 bp), registered data of the standard strains of CBS, as well as those of 15 strains found by BLAST search to have high homology, were used. As a result of analysis by a neighbor-joining method (Fig. 7), it was found that strain F1476, together with *F. incarnatum,* was located outside the clade of *F. equiseti.* Also from the result of analysis by a maximum parsimony method (Fig. 8), the strain F1476 was included in the clade of *F. incarnatum.*

For the phylogenetic analysis of the Intergenic spacer (IGS) region (2358 bp), registered data of the standard strain of CBS, as well as those of 6 strains found by BLAST search to have high homology, were used. As a result of analysis by a neighbor-joining method (Fig. 9), it was found that the strain F1476, together with *F. incarnatum,* was located outside the clade of *F.equiseti.* Also from the result of analysis by a maximum parsimony method (Fig. 10), the strain F1476 was included in the clade of *F. incarnatum.*

### Confirmation of production of compound 1 by strain F1476 and CBS standard strains

As a result of LC/MS analysis, a peak whose M+H value and RT coinciding with those of a standard product of compound 1, was observed in mass chromatogram of sample solutions obtained by culturing strain F1476 and *F. incarnatum* CBS 678.77. However, compound 1 was not detected (ND) from the culture extracts of 5 standard strains of CBS excluding *F. incarnatum* CBS 678.77. In addition, the amount of production of compound 1 by *F. incarnatum* CBS 678.77 was less than the quantification limit (tr). The results are summarized in Table 11.

**Table 11: Productivity for compound 1**

| Strain | Compound 1 (mg/L) |
|---|---|
| *F. chlamydosporum* CBS 635.76 | ND |
| *F. incarnatum* CBS 678.77 | tr |
| *F. incarnatum* CBS 145.44 | ND |
| *F. equiseti* CBS 107.07 | ND |
| *F. equiseti* CBS 193.60 | ND |
| *F. equiseti* CBS 307.94 | ND |
| F 1476 | 93 |

Physicochemical properties of compound 1
Molecular weight: 659
ESI (LC/MS positive mode) m/z = 660 (M+H)
RT of compound 1 under analysis condition 5: 22.3 ± 0.1 min

As a result of TOF-MS analysis, the molecular formula presented in Table 12 was obtained. This molecular formula was identical to that of compound 1.

**Table 12: Molecular formula of compound in sample solution**

| Formula | Calculated m/z (amu) | PMM Error |
|---|---|---|
| C₃₆H₅₄NO₁₀ | 660.3742 | 0.7208 |

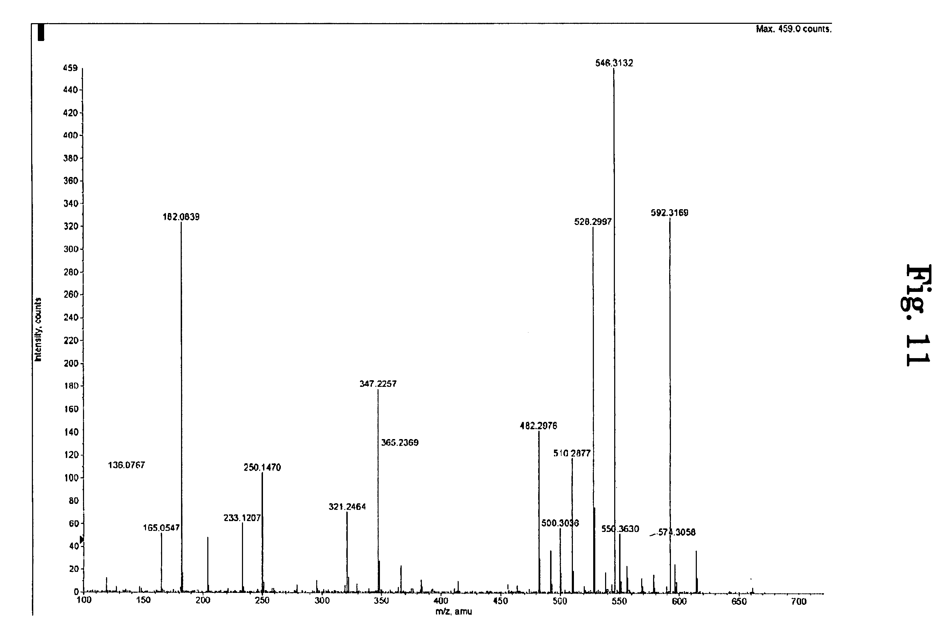

The result of MS/MS analysis was shown in Fig. 11. It was confirmed that the result was coincident with the spectrum of compound 1.

As shown above, *F. incarnatum* CBS 678.77 was found to produce compound 1.

### Discussion and Identification

Strain F1476 had a very slow growth rate, and either formed mucous, velvet-like, bright orange-colored colonies (wet part), or formed colonies mainly formed of hyphae having rigid and irregular appearance (fast part), on PDA. When subculture was continued, there was a tendency that the properties of the two parts gradually segregated, resulting in acceleration of growth. These clones showing visible differences were isolated, and the homology of nucleotide sequences of the ITS region and the TEF 1-α region was investigated and these sequences were perfectly identical. Thus, these clones were considered to be molecular phylogenetically identical.

From the fact that strain F1476 had a slow growth rate and formed orange-colored flat colonies, section Eupionnotes of *F. merismoides, Fusarium aquaeductuum* (Rabenh. & Radlk.) Sacc. or the like could be selected as candidates. However, based on the fact that strain F1476 formed polyphialides under microscopic observation, the results obtained by BLAST searching from the homology of the nucleotide sequence of ITS region, and performing a phylogenetic analysis by a neighbor-joining method and a maximum parsimony method (Figs. 5 and 6), and the fact that strain F1476 was distant from *F. merismoides* or the like, but forms a clade with *F. equiseti* and *F. incarnatum* (bootstrap value: 100), it was denied at least that the strain F1476 was Section Eupionnotes. Subsequently, a phylogentic analysis was performed using the TEF 1-α region (677 bp), and an analysis by a neighbor-joining method was performed (Fig. 7). As a result, it was found that strain F1476, together with *F. incarnatum,* was located outside the clade of *F. equiseti.* In particular, it was found that the strain F1476 was most closely related to *F. incarnatum* CBS 678.77 (bootstrap value: 66). Moreover, also from the results of an analysis by a maximum parsimony method using the nucleotide sequence of the IGS region (2358 bp) (Fig. 10), it was found that strain F1476 was included in the clade of *F. incarnatum,* and was most closely related to *F. incarnatum* CBS 678.77.

Meanwhile, as strain F1476, Fusarium forming the polyphialides that are formed by some conidium-forming cells extending to make a plurality of conidium-forming sites, or forming mesoconidia-forming cells, include *F. avenaceum* (Fr.) Sacc., *F. chenopodinum* (Thum.) Sacc., F. chlamydosporum Wollenw. & Reinking, *F. incarnatum (= F. pallidoroseum* (Cke) Sacc., *F. semitectum* Berk. & Ravenel), *F. sporotrichioides* Sherb, *F. subglutinans* (Wollenw. & Reinking) P.E. Nelson, Toussoun & Marasas (Pascoe 1990). However, based on the features and the like that the conidia are conspicuously small, the apexes of conidia spindle, or the hyphae or the reverse side dye becomes reddish purple, it was denied that the strain F1476 was something other than *F. incarnatum,* and this was not contradictory to the molecular phylogenetic data. Furthermore, *F. equiseti* which is considered to be closely related on the basis of the nucleotide sequences of the TEF 1-α region and IGS region, does not form polyphialides, and the apexes of conidia characteristically extend such that the length of a quadriseptated or more septated conidium exceeds 40 µm. Thus, *F. equiseti* is clearly different from strain F1476. From the above-discussed properties for culture, morphological features, molecular phylogenetic results and the productivity for compound 1, despite the fact that growth is particularly slow, the present fungal strain F1476 was identified with *Fusarium incarnatum* (Roberge) Saccardo.

*F. incarnatum* is considered as *F. semitectum* according to Gerlach and Nirenberg (1982), and two variants, namely, var. semitectum and *F. semitectum* var. majus Wollenw are known. The latter is characterized in having many septa (0 to 9 septa) in the conidium, and thus the size increases along therewith. From this point of view, strain F1476 is closer to *F. semitectum* var. semitectum. Booth and Sutton (1984) considered the two variants of *F. semitectum* to be identical, and used the name of *F. pallidoroseum.* However, Nirenberg (1990) reported that *F. incarnatum* corresponds to *F. semitectum* var. majus. Further, as previously mentioned, there were two variants of *F. semitectum* in history, but Pascoe (1990) integrated the two variants into one species named as *F. pallidoroseum.* Then, Nirenberg (1990) acknowledged the integration, but since *F. incarnatum* has an earlier priority in terms of nomenclature, *F. pallidoroseum* was provided as a synonym. With the nomenclature problem concerning whether or not the two variants should be acknowledged, there is still room for discussion whether to take *F. incarnatum* or to take *F. pallidoroseum* (Khoa et al., 2004). Among the standard fungal strains of CBS used in this comparison, phylogenetically the most closely related is CBS 678.77. This fungal strain is a strain separated from the earth of Tsu city, Mie prefecture, and described as Pseudo*Fusarium semitectum* (Berk. & Ravenel) Matsush. by Matsushima (1975). For other standard strains of *F. incarnatum,* CBS 161.25 originates in Australia, and the place of origin of CBS 145.44 is not known but cannot be considered to be Japan. Thus, it is reasonable that the strain F1476 is likewise phylogenetically very closely related to the *F. incarnatum* CBS 678.77 originating in Japan. This is also supported by the fact that the strain F1476 produces compound 1 as in the case of CBS 678.77.

The following literatures were referred to for the present identification:
Aoki T and O'Donnell K. 1998. Fusarium kyushuense sp. nov. from Japan. Mycoscience 39: 1-6.
Aoki T, O'Donnell K, Homma Y, and Lattanzi AR. 2003. Sudden-death syndrome of soybean is caused by two morphologically and phylogenetically distinct species within the Fusarium solani species complex - F. virguliforme in North America and F. tucumanae in South America. Mycologia 95: 660-684.
Booth C and Sutton BC. 1984. Fusarium pallidoroseum, the correct name for F. semitectum auct. Trans. Br. Mycol. Soc. 83: 702-704.
Gerlach W and Nirenberg H. 1982. The genus Fusarium - a pictorial atlas. Mitt. Biol. Bundesanst. Land- u. Forstwirtsch. Berlin-Dahlem 209:1-406.
Khoa LV, Hatai K and Aoki T. 2004. Fusarium incarnatum isolated from black tiger shrimp, Penaeus monodon Fabricius, with black gill disease cultured in Vietnam. J Fish Diseases 27: 507-515.
Kosiaka EB, Holst-Jensena A, Rundbergeta T, Jaenb MTG and Torpa M. 2005. Morphological, chemical and molecular differentiation of Fusarium equiseti isolated from Norwegians. International Journal of Food Microbiology. 195-206.
Matsushima K. 1975. Icones Microfungorum A Matsushima Lectorum. published by the author, Kobe.
Nirenberg HI. 1990. Recent advances in the taxonomy of Fusarium. Studies in Mycology. 32: 91-101.
O'Donnell K, Kistler HC, Cigelnik E and Ploetz RC. 1998. Multiple evolutionary origins of the fungus causing Panamadisease of banana: concordant evidence from nuclear and mitochondrial gene genealogies. Proceedings of the National Academy of Science, USA 95: 2044-2049.
Pascoe IG. 1990. Fusarium morphology I: identification and characterization of a third conidial type, the mesoconidium. Mycotaxon 37: 121-160.
Swofford DI. 2000. PAUP, phylogenetic analysis using parsimony. ver 4.0 beta10, Sinauer Associates Inc. Publishers, Sutherland, MA.
White TJ, Bruns T, Lee S and Taylor JW. 1990. Amplification and direct sequencing of fungal ribosomal RNA genes for phylogenetics. In PCR Protocols: A guide to methods and application, Academic Press, New York, 315-322.

## Claims

1. A method of biologically producing a compound represented by the formula (1): 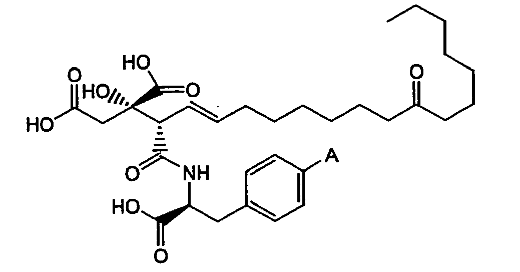 wherein A represents a hydrogen atom, a straight or branched alkyl group having 1 to 8 carbon atoms, a straight or branched alkenyl group having 2 to 8 carbon atoms, a straight or branched alkynyl group having 2 to 8 carbon atoms, -OR¹, an aryl group which may be substituted, or a heteroaryl group which may be substituted;
wherein R¹ represents a hydrogen atom, a straight or branched alkyl group having 1 to 8 carbon atoms, a straight or branched alkenyl group having 2 to 8 carbon atoms, a straight or branched alkynyl group having 2 to 8 carbon atoms, a cycloalkyl group having 3 to 8 carbon atoms, an aryl group which may be substituted, a heteroaryl group which may be substituted, an aralkyl group which may be substituted, or a heteroarylalkyl group which may be substituted,
or a pharmaceutically acceptable salt thereof, the method comprising adding an amino acid derivative represented by the formula (3): 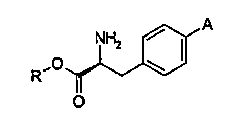 wherein A has the same meaning as defined for the compound of the above formula (1); and R represents a hydrogen atom, a straight or branched alkyl group having 1 to 8 carbon atoms, a straight or branched alkenyl group having 2 to 8 carbon atoms, or a straight or branched alkynyl group having 2 to 8 carbon atoms,
or a salt thereof into a fungal mycelium or a culture medium/culture broth of a filamentous fungal strain having an ability to produce a compound represented by the formula (2) : 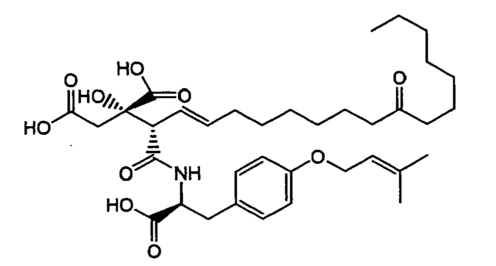 to thereby cause the fungal strain to directly produce the compound of the above formula (1).

2. The production method according to claim 1, wherein R in the amino acid derivative of the formula (3) or a salt thereof is a methyl group.

3. The production method according to claim 1 or 2, wherein the amino acid derivative of the formula (3) or a salt thereof is a hydrochloride salt of the compound of the formula (3).

4. The production method according to any one of claims 1 to 3, wherein A is a phenyl group which may be substituted, or a straight or branched alkynyloxy group having 2 to 8 carbon atoms.

5. The production method according to any one of claims 1 to 4, wherein the fungal strain producing the compound of the formula (2) is strain F1476, or a strain which is taxonomically or genetically equivalent to said strain, or a mutant strain thereof.

6. The production method according to any one of claims 1 to 4, wherein the fungal strain producing the compound of the formula (2) is a strain having at least one feature selected from the following a) and b), or a mutant strain thereof:
a) the strain belongs to *Fusarium incarnatum,* or represents a form equivalent to that of *Fusarium incarnatum*; and
b) the nucleotide sequence of ITS region of the strain is 99% or more homologous to that of *Fusarium* sp. strain F1476.

7. The production method according to any one of claims 1 to 4, wherein the fungal strain producing the compound of the formula (2) is *Fusarium* sp. strain F1476, or a mutant strain thereof.

8. Use of an amino acid derivative represented by the formula (3): 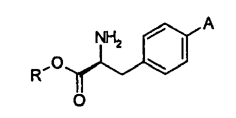 wherein A has the same meaning as defined for the compound of the formula (1) in claim 1; and R represents a hydrogen atom, a straight or branched alkyl group having 1 to 8 carbon atoms, a straight or branched alkenyl group having 2 to 8 carbon atoms, or a straight or branched alkynyl group having 2 to 8 carbon atoms,
or a salt thereof as an additive for culture medium.

9. An amino acid derivative represented by the formula (3'): 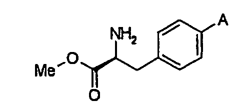 wherein A has the same meaning as defined for the compound of the formula (1) in claim 1,
or a salt thereof.

10. An additive for culture medium containing an amino acid derivative represented by the formula (3): 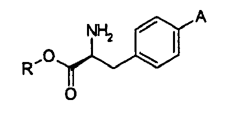 wherein A has the same meaning as defined for the compound of the formula (1) in claim 1; and R represents a hydrogen atom, a straight or branched alkyl group having 1 to 8 carbon atoms, a straight or branched alkenyl group having 2 to 8 carbon atoms, or a straight or branched alkynyl group having 2 to 8 carbon atoms,
or a salt thereof.
